# FASCICULE DE BREVET EUROPEEN

(11) **EP 3 350 192 B1**
(45) Date de publication et mention de la délivrance du brevet: **07.08.2019**
(21) Numéro de dépôt: 16787487.4
(22) Date de dépôt: 15.09.2016
(51) Int. Cl.: C07H 15/04, A61K 31/7028, A61P 35/00

(54) **COMPOSÉS POLYSACCHARIDES MULTI-FONCTIONNALISÉS ET LEUR UTILISATION POUR CIBLER LE RÉCEPTEUR DU MANNOSE 6-PHOSPHATE CATION-INDÉPENDANT**
MULTIFUNKTIONALISIERTE POLYSACCHARIDVERBINDUNGEN UND VERWENDUNG DAVON ZUR ABZIELUNG AUF DEN KATIONUNABHÄNGIGEN MANNOSE-6-PHOSPHAT-REZEPTOR
MULTI-FUNCTIONALIZED POLYSACCHARIDE COMPOUNDS AND USE THEREOF FOR TARGETING THE CATION-INDEPENDENT MANNOSE 6-PHOSPHATE RECEPTOR

(30) Priorité: 18.09.2015 FR 1558806
(43) Date de publication de la demande: 25.07.2018
(73) Titulaire: Nanomedsyn, 34093 Montpellier cedex 5 (FR); Centre National de la Recherche Scientifique, 75016 Paris (FR); Université de Montpellier, 34090 Montpellier (FR)
(72) Inventeur: MORERE, Alain, 34980 Saint Gely du Fesc (FR); DA SILVA, Afitz, Montréal QC H2X 0B1 (CA); BOUFFARD, Elise, 86550 Mignaloux Beauvoir (FR); EL CHEIKH, Khaled, 34090 Montpellier (FR); DURAND, Jean-Olivier, 34250 Palavas les Flots (FR); MAYNADIER, Marie, 34800 Ceyras (FR); BASILE, Ilaria, 34280 Saint Gely du Fesc (FR)
(74) Mandataire: Cabinet Plasseraud
(86) Numéro de dépôt international: PCT/FR2016/052339
(87) Numéro de publication internationale: WO 2017/046535

(56) Documents cités:
- WO-A1-2011/000958

## Description

La présente invention concerne de nouveaux composés polysaccharides multifonctionnalisés, et plus particulièrement des composés di-, tri- ou tétra-mannosidiques multi-fonctionnalisés. L'invention concerne également le procédé de préparation desdits composés, et leur utilisation pour cibler le récepteur du mannose 6-phosphate cation-indépendant (RM6P-CI).

Le récepteur du mannose 6-phosphate cation-indépendant (RM6P-CI) est un récepteur ubiquitaire qui est présent à la fois dans le cytoplasme et à la membrane des cellules. Son rôle principal est d'assurer le transport des enzymes lysosomales nouvellement synthétisées depuis le réseau trans-golgien jusqu'aux lysosomes où elles sont actives. Il participe également à l'endocytose des enzymes lysosomales secrétées par les cellules. Cette endocytose par le RM6P-CI permet l'internalisation de composés portant des résidus mannose 6-phosphate (M6P) au sein de la cellule et leur adressage vers les lysosomes.

Le RM6P-CI joue donc un rôle crucial dans le traitement des maladies de surcharge lysosomale (maladie rare), et plus particulièrement pour l'enzymothérapie substitutive. L'enzymothérapie substitutive est une thérapie utilisée avec succès pour traiter ces maladies rares qui se caractérisent par une déficience d'une enzyme lysosomale spécifique. Elle consiste en l'administration d'enzymes lysosomales recombinantes qui vont être dirigées vers les lysosomes via le RM6P-CI grâce aux résidus M6P présents à l'extrémité de leurs chaînes glycosylées.

De plus, le RM6P-CI est surexprimé dans certains cancers¹, ce qui en fait une cible intéressante pour l'élaboration de traitements ciblés, limitant ainsi la toxicité sur les cellules saines.

De telles thérapies peuvent être obtenues par la fonctionnalisation, par exemple, de nanoparticules encapsulant un principe actif et greffées en surface par des résidus de mannose 6-phosphate (M6P).

Cependant, l'inconvénient majeur du mannose 6-phosphate (M6P) est la sensibilité de sa fonction phosphate à l'hydrolyse par les phosphatases présentes dans tous les organes et dans le sérum. Le M6P est alors déphosphorylé et n'est plus reconnu par le RM6P-CI. La stabilité de ce marqueur de reconnaissance est donc un élément déterminant pour son utilisation dans le transport de molécules bioactives.

Les analogues isostères du M6P ne présentent pas l'inconvénient de la dégradation par des phosphatases^{2,3,4}. La ligation de ces analogues isostères du M6P à la partie oligosaccharidique des enzymes lysosomales permet d'améliorer l'efficacité de ces enzymes pour des applications en thérapie enzymatique substitutive⁵. L'utilisation de ces analogues isostères permet également un ciblage très efficace des cellules cancéreuses des tumeurs de la prostate surexprimant le RM6P-CI ainsi que leur traitement¹.

Afin d'améliorer l'affinité pour le RM6P-CI, ces mêmes analogues ont été synthétisés en série dimannosidique avec une liaison glycosidique de type α(1,2). Le disaccharide 6-P-Man-α(1,2)-Man possède une affinité pour le RM6P-CI supérieure à celle du monosaccharide M6P⁶. Le lien alpha 1,2 entre les deux résidus mannose est très important pour obtenir une bonne affinité vis-à-vis du RM6P-CI. Les liens alpha 1,3 ou alpha 1,4 ou alpha 1,6 conduisent à une affinité inférieure.

Le disaccharide 6-P-Man-α(1,2)-Man présente cependant l'inconvénient de ne pas être stable dans le sang. En effet, la fonction phosphate du disaccharide 6-P-Man-α(1,2)-Man subit l'hydrolyse par les phosphatases présentes dans le sérum. Après déphosphorylation par les hydrolases du sérum, le disaccharide formé Man-α(1,2)-Man n'est pas reconnu par le RM6P-CI qui est la cible biologique.

Il subsiste à ce jour la nécessité de trouver de nouveaux analogues isostères du M6P présentant une affinité améliorée pour le RM6P-CI, et qui en outre sont stables dans les liquides physiologiques.

L'un des buts de la présente invention est donc de proposer de nouveaux analogues isostères du M6P présentant une très grande affinité pour le RM6P-CI.

On entend par analogue isostère du M6P, un composé chimique synthétique présentant la même activité biologique que le M6P, mais une stabilité améliorée.

Un autre but de l'invention est de proposer de nouveaux analogues isostères du M6P qui soient stables dans les liquides physiologiques.

Un autre but de l'invention est de proposer de nouveaux analogues isostères du M6P présentant une affinité améliorée pour le RM6P-CI par rapport aux analogues isostères du M6P décrits dans la demande PCT/EP2010/05950⁵.

Les Inventeurs ont ainsi imaginé de nouveaux composés di-, tri- ou tétra-mannosidiques multi-fonctionnalisés, c'est-à-dire portant différents groupes fonctionnels, lesdits groupes fonctionnels étant aptes à former respectivement une (ou des) liaison(s) avec le RM6P-CI et une (ou des) liaison(s) avec un composé d'intérêt Y.

La difficulté rencontrée avec les composés proposés dans l'invention est de trouver une façon avantageuse de les synthétiser, à savoir notamment une façon qui soit simple à mettre en oeuvre, efficace (bon rendement) et non coûteuse.

Un autre but de l'invention est donc de trouver un procédé de préparation des composés di-, tri- ou tétra-mannosidiques multi-fonctionnalisés qui soit avantageux, à savoir notamment qui soit simple à mettre en oeuvre, efficace et non coûteux.

En effet, la synthèse de composés di-, tri- ou tétra-saccharidiques est plus complexe à mettre en oeuvre que celle de composés mono-saccharidiques.

La présente invention a pour objet un composé caractérisé en ce qu'il présente la formule générale (I) : 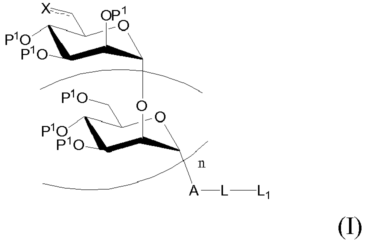 dans laquelle :
∘ n est un entier allant de 1 à 3,
∘ ------- représente une liaison simple ou une liaison double,
∘ chacun des P¹ représente indépendamment l'un de l'autre H, un groupe protecteur notamment choisi parmi le triméthylsilyle (TMS : (CH₃)₃Si-), le tertbutyldiméthylsilyle (TBDMS : tBuMe₂Si-), le benzyle (Bn : C₆H₅CH₂-), le para-méthoxybenzyle (PMB : 4-CH₃OC₆H₅CH₂-), l'ortho-nitrobenzyle (o-NO₂C₆H₅CH₂-), l'acétyle (Ac :CH₃CO-), le benzoyle (Bz : C₆H₅CO-) ou CF₃CO- (trifluoroacétyle),
∘ X représente :
   ∘∘ lorsque ------- est une liaison simple :
      ∘∘∘ le groupement phosphonate : 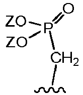
      ∘∘∘ le groupement fluoro phosphonate : 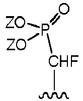
      ∘∘∘ le groupement bi-fluoro phosphonate : 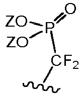
      ∘∘∘ le groupement carboxylate : 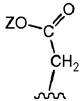
      ∘∘∘ le groupement malonate : 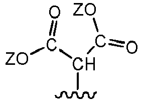
   ∘∘ lorsque ------- est une liaison double :
      ∘∘∘ le groupement phosphonate : 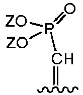
      ∘∘∘ le groupement carboxylate : 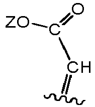 avec Z représentant indépendamment l'un de l'autre H; un alkyle en C₁-C₅, notamment choisi parmi méthyle (Me : CH₃-), éthyle (Et : C₂H₅) ou isopropyle (iPr : (CH₃)₃C-) ; le 2,2,2-trifluoroéthyle (CF₃CH₂-) ; C₆H₅CH₂- ; le phényle (Ph : C₆H₅-) ; (CH₃)₃Si- ; un métal alcalin choisi parmi Na, Li ou K ; un ammonium NH₄ ;
∘ A représente un radical divalent choisi parmi -O-, -S-, -NH-, -CH₂-,
∘ L représente :
   ∘∘ -H ; -NH₂ ; -(CH₂)ₙ₁-CH=CH₂ ou -(CH₂)ₙ₁-C≡CH avec n₁ représentant un entier allant de 0 à 4, alors dans chacun de ces cas L₁ est absent,
   ∘∘ un radical hydrocarboné divalent saturé, linéaire ou ramifié, substitué ou non substitué, ayant de 1 à 30 atomes de carbone, un radical hydrocarboné divalent insaturé, linéaire ou ramifié, substitué ou non substitué, ayant de 2 à 30 atomes de carbone,
   ∘∘ un radical hydrocarboné divalent saturé ou insaturé tel que défini ci-dessus dont un ou plusieurs groupements -CH₂-, -CH=CH- et/ou -C=C- du radical hydrocarboné saturé ou insaturé est (sont) remplacé(s) indépendamment l'un de l'autre par un groupement éther (-O-) ; amine (-NH-) ; alkylamine (-NR₁-) avec R₁ représentant un alkyle en C₁-C₅ ; thioéther (-S-) ; amide (-CO-NH-) ; carbamate (-NH-CO-O-); oxime -0-N=CH- ; acylhydrazone -CO-NH-N=CH- ; un système cyclique ou hétérocyclique, saturé ou insaturé, substitué ou non ;
∘ L₁ représente :
   -(CH₂)ₙ₁-CH=CH₂ ; -(CH₂)ₙ₁-C≡CH ; -(CH₂)ₙ₁-N₃; -(CH₂)ₙ₁-SH; -(CH₂)ₙ₁-NH₂; -(CH₂)ₙ₁-N=C=O; -(CH₂)ₙ₁-N=C=S; -(CH₂)ₙ₁-NHR₁; -(CH₂)ₙ₁-NR₁R₂; -(CH₂)ₙ₁-A₁-NH₂; -(CH₂)ₙ₁-A₁-NHR₁; -(CH₂)ₙ₁-A₁-NR₁R₂; -(CH₂)ₙ₁-NHCO-CH₂Ha1; -(CH₂)ₙ₁-COZ₁; -(CH₂)ₙ₁-A₁COZ₁; -(CH₂)ₙ₁-O-N=CH₂; -(CH₂)ₙ₁-CO-NH-N=CH₂; -(CH₂)ₙ₁-H; un système cyclique ou hétérocyclique, saturé ou insaturé, substitué ou non; un halogène choisi parmi F, Cl, Br ou I ; avec
   n₁ tel que défini ci-dessus,
   R₁ et R₂ représentant indépendamment l'un de l'autre un alkyle en C₁-C₅,
   A₁ représentant -O-, -NH-,
   Hal représentant Cl, Br ou I ;
   Z₁ représentant -OH, -OR₁, -NHR₁, -NH-NH₂, -NH-NHR₁, -NH-NR₁R₂ avec R₁ et R₂ tels que définis ci-dessus, un halogène choisi parmi F, Cl, Br ou I.

A titre d'exemples d'un radical hydrocarboné divalent saturé, linéaire ou ramifié, ayant de 1 à 30 atomes de carbone, on pourra notamment citer :
-CH₂-; -CH₂-CH₂-; -CH₂-(CH₂)ₘ-; -(CH₂)ₘ-CH(C₁-C₇)-(CH₂)ₘ-; (CH₂)ₘ-CH(C₁-C₇)-(CH₂)ₘ-CH(C₁-C₇)-(CH₂)ₘ-; -(CH₂)ₘ-C(C₁-C₇)₂-(CH₂)ₘ; -(CH₂)ₘ-C(C₁-C₇)₂-(CH₂)ₘ-CH(C₁-C₇)-(CH₂)ₘ-; -(CH₂)ₘ-C(C₁-C₇)₂-(CH₂)ₘ-C(C₁-C₇)₂-(CH₂)ₘ-;
avec m représentant indépendamment l'un de l'autre un entier allant de 0 à 30 avec la condition que la longueur de la chaîne hydrocarbonée principale ne dépasse pas 30 atomes de carbone, et C₁-C₇ représentant un alkyle ayant de 1 à 7 atomes de carbone. A titre d'exemple d'alkyle en C₁-C₇, on pourra notamment citer le méthyle, l'éthyle, le propyle, l'isopropyle, le butyle, l'isobutyle, le pentyle, l'hexyle, l'heptyle.

Un radical hydrocarboné divalent insaturé, linéaire ou ramifié, ayant de 2 à 30 atomes de carbone, désigne un radical hydrocarboné comportant une ou plusieurs doubles liaisons carbone-carbone et/ou une ou plusieurs triples liaisons carbone-carbone. A titre d'exemples d'un radical hydrocarboné divalent insaturé on pourra notamment citer :
- CH=CH- ; -(CH₂)ₘ-CH=CH-(CH₂)ₘ- ;
- (CH₂)ₘ-CH=CH-(CH₂)ₘ-CH=CH-(CH₂)ₘ- ;
- (CH₂)ₘ-CH=CH-CH(C₁-C₇)-(CH₂)ₘ- ;
- (CH₂)ₘ-CH(C₁-C₇)-(CH₂)ₘ-CH=CH-CH(C₁-C₇)-(CH₂)ₘ- ;
- (CH₂)ₘ-CH(C₁-C₇)-CH=CH-(CH₂)ₘ- ;
- (CH₂)ₘ-CH(C₁-C₇)-CH=CH-(CH₂)ₘ-CH=CH-(CH₂)ₘ- ;
- (CH₂)ₘ-CH=CH-C(C₁-C₇)₂-(CH₂)ₘ- ;
- (CH₂)ₘ-CH=CH-C(C₁-C₇)₂-(CH₂)ₘ-C(C₁-C₇)₂-(CH₂)ₘ-CH=CH ;
- (CH₂)ₘ-CH=CH-C(C₁-C₇)₂-(CH₂)ₘ-CH(C₁-C₇)-(CH₂)ₘ-CH=CH ;
- C≡C-; -(CH₂)ₘ-C≡C-(CH₂)ₘ-; -(CH₂)ₘ-C=C-(CH₂)ₘ-C=C-(CH₂)ₘ- ;
- (CH₂)ₘ-CH=CH-CH(C₁-C₇)-(CH₂)ₘ-C≡C-(CH₂)ₘ-CH(C₁-C₇)- ;
- (CH₂)ₘ-CH=CH-C(C₁-C₇)₂-(CH₂)ₘ-C≡C-(CH₂)ₘ-C(C₁-C₇)₂-(CH₂)ₘ- ;
- (CH₂)ₘ-C=C-CH(C₁-C₇)-(CH₂)ₘ-C≡C-(CH₂)ₘ-CH(C₁-C₇)-(CH₂)ₘ ;
- (CH₂)ₘ-C≡C-C(C₁-C₇)₂-(CH₂)ₘ-CH=CH-(CH₂)ₘ-CH(C₁-C₇)- ;
- (CH₂)ₘ-CH=CH-C(C₁-C₇)₂-(CH₂)ₘ-CH(C₁-C₇)-(CH₂)ₘ-C≡C-CH=CH-(CH₂)ₘ- ;
avec m tel que défini précédemment.

Selon l'invention, le terme « système » cyclique ou hétérocyclique désigne, selon le cas, le radical monovalent ou divalent provenant d'un composé cyclique ou hétérocyclique, saturé ou insaturé, en tant que tel.

Ainsi lorsque L₁ représente un système cyclique ou hétérocyclique, il s'agira plus précisément d'un radical monovalent provenant d'un composé cyclique ou hétérocyclique, saturé ou insaturé.

Lorsque L représente un radical hydrocarboné saturé ou insaturé dont un ou plusieurs groupements -CH₂-, -CH=CH- et/ou -C=C- est (sont) remplacé(s) indépendamment l'un de l'autre par un système cyclique ou hétérocyclique, saturé ou insaturé, alors ledit système cyclique ou hétérocyclique est un radical divalent provenant d'un composé cyclique ou hétérocyclique, saturé ou insaturé.

A titre d'exemple d'un composé cyclique ou hétérocyclique, saturé ou insaturé, on pourra notamment citer l'azétidine, l'oxétane, le thiétane, le pyrrole, le pyranose, le furanose, le furane, la pyrroline, le tétrahydrofurane, le thiophène, le tétrahydrothiophène, le pyrazole, l'imidazole, l'oxazole, l'isoxazole, la pyrazoline, l'imidazoline, la pyrazolidine, l'imidazolidine, le dioxolane, le thiazole, l'isothiazole, la thiazolidine, l'isoxazolidine, le triazole, l'oxadiazole, le thiadiazole, le thiosuccinimide, le tétrazole, la pyridine, le naphthyridine, le phtalimide, le pyrane, le dihydro-pyrane, la pipéridine, la pyridazine, le pyridinium, la pyrimidine, la purine, la pyrazine, la ptéridine, l'oxazine, la dioxine, la pipérazine, le maléimide, la morpholine, le dioxane, la thiazine, la thiomorpholine, l'oxathiane, le dithiane, la triazine, le trioxane, la thiadiazine, la dithiazine, le trithiane, le 3-cyclobutène-1,2-dione, le cyclobutane, le cyclobutène, le cyclopentane, le cyclopentène, le cyclohexane, le cyclohexène, le cycloheptane, le cycloheptène, le benzène, le toluène, le naphtalène, l'indène, l'indane, l'indolizine, l'indole, le benzofurane, l'indoline, le benzothiophène, l'indazole, le benzimidazole, le benzthiazole, la tétraline, la quinoline, le chromène, le chromane, la cinnoline, la quinazoline, la quinoxaline, la phthalazine.

Selon l'invention, et comme indiqué précédemment, ledit radical hydrocarboné divalent saturé ou insaturé, linéaire ou ramifié, et ledit système cyclique ou hétérocyclique, saturé ou insaturé, pourront en outre être substitués par un ou plusieurs substituants choisis parmi alkyle en C₁-C₇, alcényle en C₂-C₇, alcynyle en C₂-C₇, aryle, ou par des groupes fonctionnels.

A titre d'exemple de groupes fonctionnels on pourra notamment citer des fonctions alcool, amine, amide, cétone, ester, éther, thioéther ou acide carboxylique.

Le terme alcényle en C₂-C₇ désigne un radical hydrocarboné, linéaire ou ramifié, ayant 2 à 7 atomes de carbone, et comportant une ou plusieurs doubles liaisons carbone-carbone.

Le terme alcynyle en C₂-C₇ désigne un radical hydrocarboné, linéaire ou ramifié, ayant 2 à 7 atomes de carbone, et comportant une ou plusieurs triples liaisons carbone-carbone.

Le terme aryle désigne un système hydrocarboné aromatique mono-, bi- ou tricyclique, ayant de 6 à 18 atomes de carbone. A titre d'exemple on peut citer le phényle (C₆H₅), le benzyle (C₆H₅CH₂), le phénéthyle (C₆H₅CH₂CH₂), le tolyl (C₆H₄CH₃), le xylyle (C₆H₃(CH₃)₂), le benzylidène (C₆H₅CH=CH), le benzoyle (C₆H₅CO), le biphényle (ou diphényle) (C₁₂H₉), le naphtyle (C₁₀H₇).

Selon un mode de réalisation de l'invention, dans le composé de formule (1) tel que défini ci-dessus :
L représente :
   ∘ -NH₂, -(CH₂)ₙ₁-CH=CH₂ ou -(CH₂)ₙ₁-C≡CH, avec n₁ tel que défini ci-dessus (entier allant de 0 à 4), alors dans chacun de ces cas L₁ est absent,
   ∘ un radical hydrocarboné divalent saturé, linéaire ou ramifié, substitué ou non substitué, ayant de 1 à 10 atomes de carbone, un radical hydrocarboné divalent insaturé, linéaire ou ramifié, substitué ou non substitué, ayant de 2 à 10 atomes de carbone,
   ∘ un radical hydrocarboné divalent saturé ou insaturé tel que défini ci-dessus, dont un ou plusieurs groupements -CH₂-, -CH=CH- et/ou -C=C- du radical hydrocarboné saturé ou insaturé est(sont) remplacé(s) indépendamment l'un de l'autre par :
      ∘∘ un groupement -O- ; -NH- ; -S-; -CO-NH- ; -NH-CO-O- ; et/ou
      ∘∘ un système cyclique ou hétérocyclique choisi parmi : 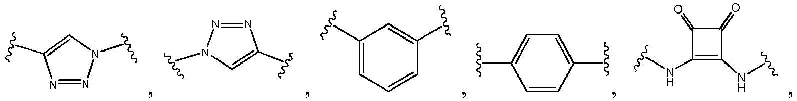 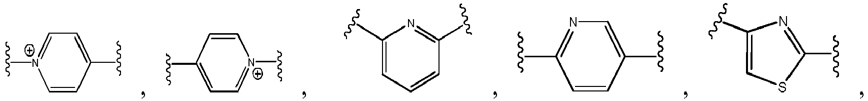 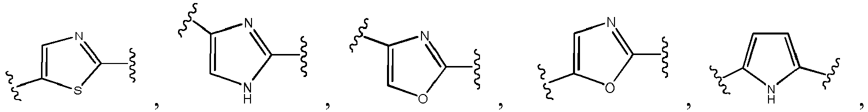 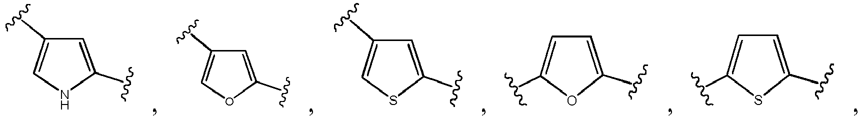 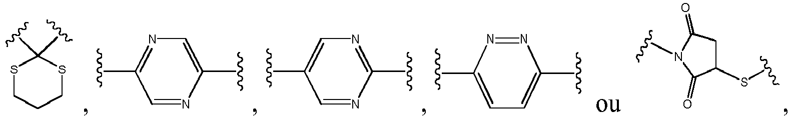
L₁ représente :
   -(CH₂)ₙ₁-CH=CH₂ ; -(CH₂)ₙ₁-C≡CH ; -(CH₂)ₙ₁-N₃; -(CH₂)ₙ₁-SH ; -(CH₂)ₙ₁-NH₂ ; -(CH₂)ₙ₁-N=C=O ; -(CH₂)ₙ₁-N=C=S ; -(CH₂)ₙ₁-O-NH₂ ; -(CH₂)ₙ₁-NHCO-CH₂Hal ; -(CH₂)ₙ₁-COOH ; -(CH₂)ₙ₁-COOR₁ ; -(CH₂)ₙ₁-CO-NH-NH₂ ; -(CH₂)ₙ₁-H ; un halogène choisi parmi F, Cl, Br ou I ;
   ∘ un système cyclique ou hétérocyclique choisi parmi : 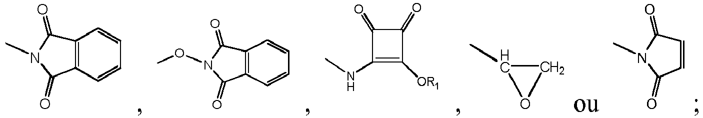 avec n₁, R₁ et Hal tels que définis ci-dessus.

A titre d'exemples de composés de formule (I) tels que définis ci-dessus, on pourra citer ceux pour lesquels :
∘ le substituant L est choisi parmi :
   ∘∘ -CH=CH₂, -C≡CH ou -NH₂ alors dans chacun de ces cas L₁ est absent,
   ∘∘ -CH₂- ; -CH₂-CH₂- ; -(CH₂)₃- ; -(CH₂)₄- ; -(CH₂)₅- ; -CH₂-CH₂-O-CH₂-CH₂- ; -(CH₂-CH₂-O)₂-CH₂-CH₂- ; -CH₂-CH₂-S-CH₂-CH₂- ; -CH₂-CH₂-S-CH₂-CH₂-O-CH₂-CH₂- ; 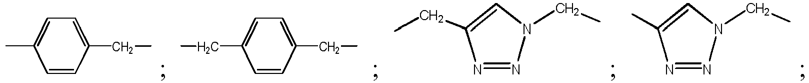 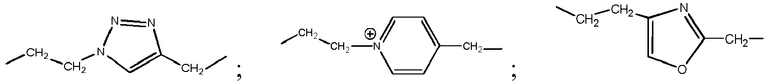 ou 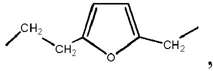 et
∘ le substituant L₁ est choisi parmi :
   ∘∘ -CH=CH₂ ; -C=CH ; -N₃ ; -SH; -NH₂ ; -N=C=O ; -N=C=S; -O-NH₂ ; -NHCO-CH₂Cl ; -COOH ; -COOR₁ ; -CO-NH-NH₂, un halogène choisi parmi F, Cl, Br ou I,
   ∘∘ un système cyclique ou hétérocyclique choisi parmi : 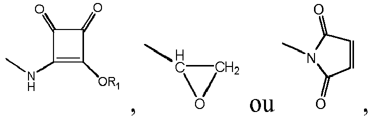 avec R₁ tel que défini ci-dessus.

Un composé de formule (I) dans lequel n est égal à 1 est un disaccharide, et plus particulièrement un di-mannoside multi-fonctionnalisé répondant à la formule :

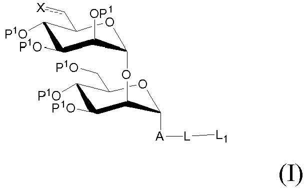

Un composé de formule (I) dans lequel n est égal à 2 est un trisaccharide, et plus particulièrement un tri-mannoside multi-fonctionnalisé répondant à la formule :

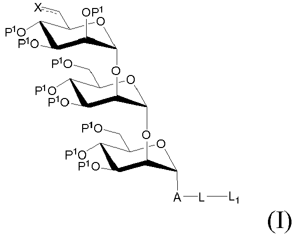

Un composé de formule (I) dans lequel n est égal à 3 est un composé tétrasaccharide, et plus particulièrement un tétra-mannoside multi-fonctionnalisé répondant à la formule :

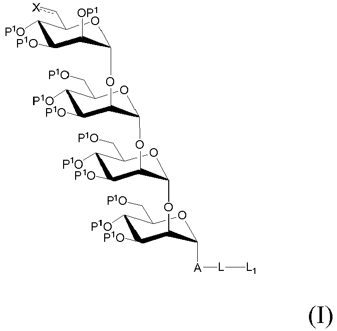

L'invention concerne également un procédé de préparation d'un composé de formule (I) tel que défini ci-dessus, caractérisé en ce que le composé de départ utilisé est un composé répondant à la formule (II) : 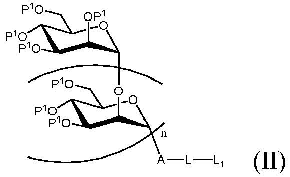 dans laquelle P¹, n, A, L et L₁ sont tels que définis ci-dessus.

Ces composés sont très avantageux car ils permettent notamment de préparer des composés de formule (I) en un nombre limité d'étapes, et avec un bon rendement.

L'invention a donc également pour objet un composé répondant à la formule générale (II) : 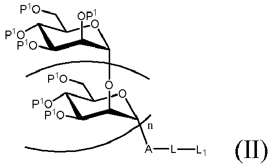 dans laquelle P¹, n, A, L et L₁ sont tels que définis ci-dessus.

La préparation du composé de formule (II) s'effectue par réaction entre un accepteur de glycoside (IV) ou (IVbis) avec un donneur de glycoside (V), selon l'un des schémas réactionnels ci-dessous : 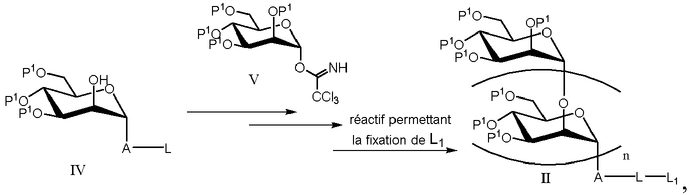 ou

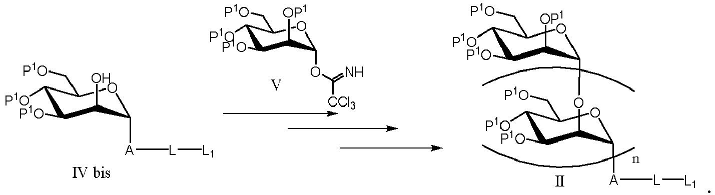

Les composés de l'invention de formule (I) sont particulièrement avantageux car ils permettent d'une part de cibler le RM6P-CI avec une très grande affinité et d'autre part car ils sont capables de former des liaisons covalentes au niveau de leur groupement LL₁ avec un grand nombre de composés d'intérêt Ⓨ.

La présente invention se rapporte également à un conjugué formé entre un composé d'intérêt Ⓨ et le composé de formule (I).

L'invention a ainsi encore pour objet un conjugué caractérisé en ce qu'il répond à la formule générale (III) : 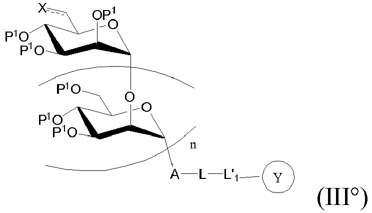
dans laquelle X, P¹, n, A et L sont tels que définis ci-dessus,
L₁' représente le substituant L₁ tel que défini ci-dessus impliqué dans une liaison covalente avec un groupement fonctionnel porté par un composé d'intérêt Ⓨ, ledit composé d'intérêt Ⓨ étant choisi parmi des enzymes, des nanoparticules, des protéines, des anticorps ou des agents cytotoxiques.

Selon un mode de réalisation de l'invention, le conjugué de formule (III) tel que défini ci-dessus présente une valeur IC₅₀ pour le récepteur du mannose 6-phosphate cation-indépendant (RM6P-CI) d'au moins 10⁻⁵ M, et de préférence allant de 10⁻⁶ à 10⁻⁹ M.

On entend par valeur IC₅₀ la concentration de composés capables d'inhiber la liaison du RM6P-CI à son ligand, le pentamannose 6-phosphate (PMP), préalablement adsorbé.

A titre d'exemples plus spécifiques du composé d'intérêt Ⓨ, on pourra citer une enzyme lysosomale ou une nanoparticule, notamment une nanoparticule de silice.

Selon un mode de réalisation de l'invention, la nanoparticule pourra incorporer un photosensibilisateur de type porphyrine neutre.

A titre d'exemples d'enzymes lysosomales, on pourra citer celles choisies parmi alpha glucosidase acide, béta-galactosidase-1 acide, sphingomyélinase acide, alpha-D-mannosidase, alpha-fucosidase, alpha-galactosidase A, alpha-glucosaminide acétyltransférase, alpha-glucosidase, alpha-L-iduronidase, alpha-N-acétylgalactosaminidase, alpha-acétylglucosaminidase, alpha-D-neuraminidase, arylsulfatase A, arylsulfatase B, bêta-galactosidase, bêta-glucuronidase, bêta-mannosidase, cathepsine D, cathepsine K, céramidase, cystinosine, facteur d'activation du ganglioside GM2, galactocérébrosidase, glucocérébrosidase, héparane sulfatase, hexosaminidase A, hexosaminidase B, hyaluronidase, iduronate-2-sulfatase, LAMP2, lipase acide lysosomale, N-acétylglucosamine-1-phosphotransférase, N-acétylgalactosamine 6-sulfatase, N-acétylglucosamine-1-phosphotransférase, N-acétylglucosamine-6-sulfate sulfatase, N-aspartyl-bêta-glucosaminidase, palmitoyl-thioestérase-1, phosphatase acide, protéine protectrice/cathepsine A (PPCA), sialine, tripeptidyl-peptidase 1.

Lesdites enzymes sont obtenues par génie génétique dans des systèmes de production permettant l'obtention de protéines recombinantes biologiquement actives et fonctionnelles. Par exemple, pour la production de glycoprotéines lysosomales, le système baculovirus/cellules d'insecte sf9 est utilisé.

L'invention concerne encore l'utilisation d'au moins un composé de formule (I) tel que défini ci-dessus, pour former au moins une liaison covalente avec au moins un groupement fonctionnel porté par un composé d'intérêt Ⓨ, ledit composé d'intérêt Ⓨ étant choisi parmi des enzymes, des nanoparticules, des protéines, des anticorps ou des agents cytotoxiques.

Selon un mode de réalisation avantageux de l'invention, n' composés de formule (I) peuvent réagir avec n' groupement(s) fonctionnel(s) porté(s) par ledit composé d'intérêt Y, avec n' étant un entier allant de 1 à 1000, et de préférence de 1 à 100, de façon à former n' liaisons covalentes.

L'invention concerne ainsi également l'utilisation de n' composé(s) de formule (I) pour former n' liaison(s) covalente(s) avec n' groupement(s) fonctionnel(s) du composé d'intérêt Ⓨ, avec n' tel que défini ci-dessus.

L'invention a encore pour objet l'utilisation de n' composé(s) de formule (I) telle que définie ci-dessus pour former le conjugué de formule générale (IIIbis) : 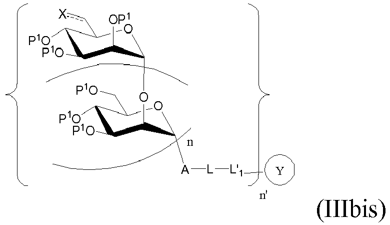 dans laquelle P¹, X, n, n', A, L, L' 1 et Ⓨ sont tels que définis ci-dessus.

Lorsque n' est un entier égal à 1, alors le conjugué de formule générale (III) et le conjugué de formule générale (IIIbis) sont identiques.

Le terme conjugué de formule générale (III) se réfère à un polysaccharide de formule (I) lié de manière covalente au niveau de son groupement L₁ avec un groupement fonctionnel du composé d'intérêt Ⓨ.

Le terme « conjugué de formule générale (IIIbis) » se réfère, lorsque n' n'est pas égal à 1, à au moins deux polysaccharide(s) de formule (I) liés de manière covalente avec au moins deux groupements fonctionnels du composé d'intérêt Ⓨ.

Par exemple, lorsque le composé d'intérêt Ⓨ est une nanoparticule, le conjugué (IIIbis) pourra comprendre 100 polysaccharides de formule (I) formant respectivement 100 liaisons covalentes avec 100 groupements fonctionnels portés par la nanoparticule.

Le groupement LL₁ des composés (I) de l'invention est tel que défini précédemment et est capable de se lier par une liaison covalente, directement ou après activation, à au moins l'une des fonctions naturellement présentes ou artificiellement introduites sur le composé d'intérêt Ⓨ.

Ainsi que mentionné précédemment par la définition des substituants L et L₁, le substituant LL₁ des composés (I) de l'invention pourra notamment comprendre un groupement réactif choisi parmi acide carboxylique et ses sels, chlorure d'acide, ester (ester d'alkyle, ester de p-nitrophényle, ester de succinimidyle, ester de sulfosuccinimidyl etc.), azido (acyl azide, azidonitrophenyl etc.), hydrazide, 3-acyl-1,3-thiazolidine-2-thione, amine substituée ou non, O-alkyloxyamine, ammonium quaternaire, isocyanate, isothiocyanate, hydrazine, phtalimido, maléimide, halogénoacétamide, monochlorotriazine, dichlorotriazine, pyridine mono- ou dihalogénée, thiol, chlorure de sulfonyle, vinylsulfone, disulfure, hydroxyle, époxyde, ou imidazolyle.

Dans un mode de réalisation particulier, le substituant LL₁ comprend un groupement carbonyle réactif choisi parmi acylhydrazide, hydrazine ou O-alkyloxyamine. La réaction entre ledit acylhydrazide, hydrazine ou O-alkyloxyamine avec un groupement carbonyle du composé d'intérêt Ⓨ conduit respectivement à une liaison acylhydrazone, hydrazone ou une liaison oxime. Ce type de groupements chimiques est généralement utile pour lier des glycoprotéines (composé d'intérêt Ⓨ): les groupes carbonyle (soit naturellement présents ou induits par l'oxydation de fonctions hydroxyles des chaînes glycosyles de la glycoprotéine) disponibles sur les fragments d'oligosaccharide de la glycoprotéine sont mis à réagir avec les groupements carbonyles réactifs des composés (I) de l'invention.

A titre d'exemples plus particuliers :
- des fonctions carbonyles du composé d'intérêt Ⓨ peuvent réagir avec des fonctions O-alkylamine ou acylhydrazide du composé (I) (à savoir L₁= -O-NH₂ ou-CO-NH-NH₂) afin de conduire à la formation de liens oxime ou acylhydrazone ;
- des fonctions amine du composé d'intérêt Ⓨ peuvent réagir avec des fonctions squarate d'éthyle ou ester activé du composé (I) (à savoir par exemple L₁= 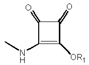 ou -COOH activé avec N-hydroxysuccinimide (NHS) ou avec hydroxybenzotriazole (HOBt)) afin de conduire à la formation de liens squarate ou de liens amide ;
- des fonctions thiol du composé d'intérêt Ⓨ peuvent réagir avec des fonctions thiol, disulfure, maléimide du composé (I) (à savoir L₁= -SH, -S-S-R₁ avec R₁ alkyle en C₁-C₅, 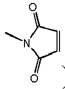 ) afin de conduire à la formation de liens disulfures ou thiolènes ;
- des fonctions alcynes ou azotures du composé d'intérêt Ⓨ peuvent réagir respectivement avec une fonction azoture du composé (I) (à savoir L₁= N₃) ou alcyne (à savoir L₁= -C=CH) afin de conduire à la formation d'un triazole (ce qui implique une réaction de cycloaddition entre l'azoture et l'alcyne).

Selon l'invention, le choix du substituant LL₁, du composé (I) va dépendre de la nature du composé d'intérêt Ⓨ et des groupements fonctionnels portés par ce dernier.

En effet, l'homme du métier comprend aisément que la longueur du substituant LL₁ augmentera avec l'encombrement stérique associé au composé d'intérêt Ⓨ qui doit être lié.

Par exemple, si le composé d'intérêt Ⓨ est une protéine ou glycoprotéine, alors un substituant LL₁ ne comportant pas plus de 2 ou 3 atomes consécutifs sera suffisant pour assurer une affinité satisfaisante entre le composé (I) et le RM6P-CI.

En revanche, si le composé d'intérêt Ⓨ est une protéine ou une nanoparticule qui provoque un encombrement stérique important à proximité des sites de liaison M6P du RM6P-CI, alors un substituant LL₁ comportant plus de 2 ou 3 atomes consécutifs sera nécessaire pour assurer une affinité satisfaisante entre le composé (I) et le RM6P-CI.

La présente invention concerne encore un procédé de préparation d'un conjugué de formule (III) tel que défini ci-dessus ou d'un conjugué de formule (IIIbis) tel que défini ci-dessus, caractérisé en que l'on fait réagir :
- au moins un groupement fonctionnel porté par un composé d'intérêt Ⓨ, ledit composé d'intérêt étant tel que défini ci-dessus, avec
- au moins un composé répondant à la formule (I) : 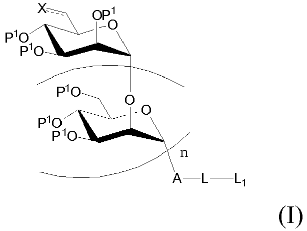 dans laquelle P¹, X, n, A, L et L₁ sont tels que définis ci-dessus.

Selon un mode de réalisation de l'invention, on fera par exemple réagir un composé de formule (I) tel que défini ci-dessus, dans lequel P¹ représente un hydrogène, et X, n, A, L, L₁ sont tels que définis ci-dessus, avec au moins un groupement fonctionnel porté par le composé d'intérêt Ⓨ tel que défini ci-dessus.

Un autre objet de l'invention porte sur un conjugué de formule (III) tel que défini ci-dessus ou un conjugué de formule (IIIbis) tel que défini ci-dessus :
- pour son utilisation dans une méthode de traitement thérapeutique du corps humain ou animal, notamment choisie parmi l'enzymothérapie substitutive, la thérapie photodynamique ou le traitement du cancer, et/ou
- pour son utilisation dans une méthode de diagnostic, notamment de diagnostic de maladies ou d'affections associées à une augmentation ou diminution de l'expression du RM6P-CI.

En effet, de par leur affinité élevée pour le RM6P-CI, les conjugués (III) et (IIIbis) de l'invention sont particulièrement utiles pour le diagnostic de maladies liées à l'expression du RM6P-CI.

Lorsque le composé d'intérêt Ⓨ est une nanoparticule incorporant un photosensibilisateur de type porphyrine, alors le conjugué de l'invention de formule (III) ou (IIIbis) sera particulièrement adapté pour la thérapie photodynamique.

A titre d'exemples de maladies pouvant être traitées à l'aide du conjugué (III) ou (IIIbis) de l'invention, on pourra citer les maladies causées par une déficience du composé d'intérêt Ⓨ dans le lysosome. Cette déficience pourra être compensée par l'administration du conjugué de l'invention, qui est capable de délivrer spécifiquement au lysosome le composé d'intérêt Ⓨ déficient.

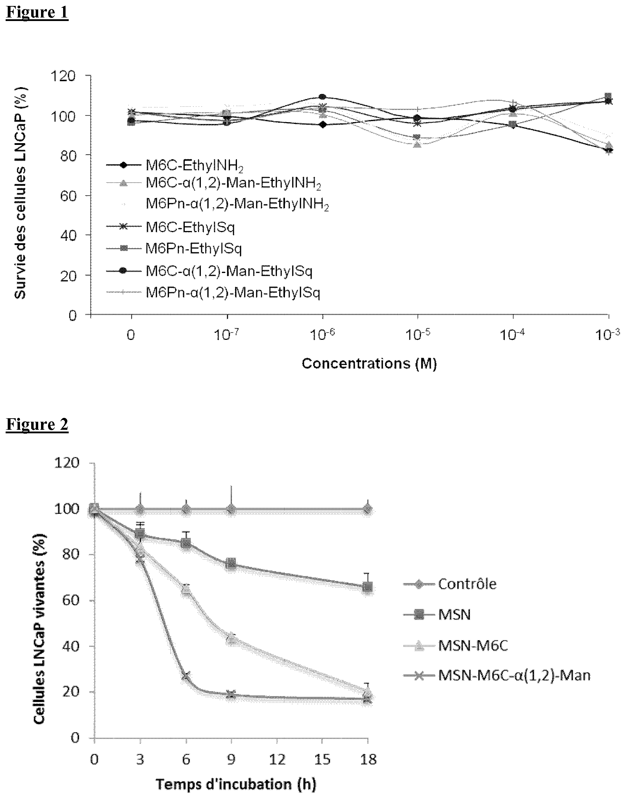

L'invention sera mieux comprise à la lumière des exemples non limitatifs et purement illustratifs suivants, et des **figures 1 et 2** suivantes.
La **figure 1** est une comparaison de la cytotoxicité des disaccharides phosphonates **18, 19** (*i.e.* M6Pn-α(1,2)-Man-Ethyl-NH₂ et M6Pn-α(1,2)-Man-EthylSq) et carboxylates **24** et **25** (*i.e.* M6C-α(1,2)-Man-Ethyl-NH₂ et M6C-α(1,2)-Man-EthylSq) de formule (I) de l'invention sur des cellules LNCaP et de leurs homologues respectifs monosaccharides.
La **figure 2** représente les effets phototoxiques, sur des cellules LNCaP, de nanoparticules de silice seules (MSN) et de nanoparticules de silice (MSN) greffées sur des analogues carboxylates monosaccharidique (M6C-EthylSq) et disaccharidique **25** (M6C-α(1,2)-Man-EthylSq) à différents temps d'incubation. Les nanoparticules greffées sur l'analogue carboxylate monosaccharidique et sur l'analogue carboxylate disaccharidique sont représentées respectivement par « MSN-M6C-EthylSq » et « MSN-M6C-α(1,2)-Man-EthylSq ».

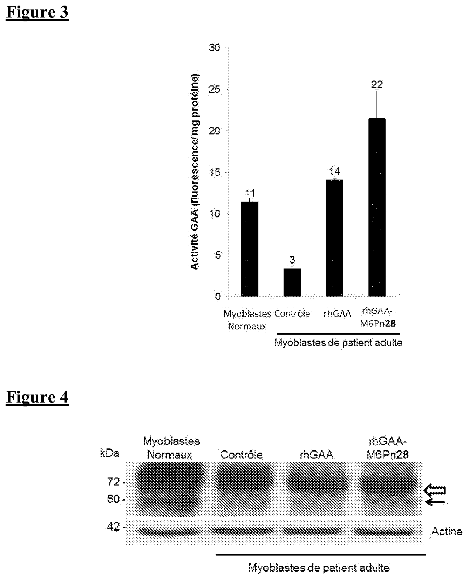

La **figure 3** indique le dosage de l'activité catalytique GAA dans des myoblastes de patients atteints de la forme adulte de la maladie de Pompe après incubation 48 h en présence de 50 nM rhGAA ou du conjugué de formule (III) de l'invention, à savoir le conjugué rhGAA-M6Pn-α(1,2)-Man-Ethyloxyamino (**28**) (dénommé rhGAA-M6Pn**28** dans la fig. 3).
La **figure 4** présente l'analyse des quantités de GAA mature par Western blot avec un anticorps anti-GAA humaine (anti-LYAG, Genetex) ou anti-actine humaine (Invitrogen) après incubation des myoblastes pendant 48 h en présence de 50 nM rhGAA ou du conjugué de formule (III) de l'invention, à savoir le conjugué rhGAA-M6Pn-α(1,2)-Man-Ethyloxyamino (**28**) (dénommé rhGAA-M6Pn**28** dans la fig. 4).

### EXEMPLE 1 :

### Synthèse de composés de formule (I)

Cet exemple décrit la préparation de disaccharides multi-fonctionnalisés répondant à la formule (I) dans laquelle n est un entier égal à 1 ; P¹ représente indépendamment l'un de l'autre H, (CH₃)₃Si- ou C₆H₅CH₂- ; ------- représente une liaison simple ou double ; X représente le groupement phosphonate ou le groupement carboxylate avec Z représentant indépendamment l'un de l'autre H, éthyle, Na ; A représente l'oxygène ; L représente -CH₂-CH₂- ; L₁ représente -Br, -NH₂, -N₃,

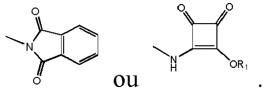

### 1) Préparation de disaccharides 9, 10, 11 et 12 répondant à la formule (II)

### a) Préparation d'un accepteur de saccharide 6 : le 3,4,6-tri-O-benzyl-α-D-mannopyranoside de 2-bromoéthyle

Le procédé de préparation d'un composé accepteur de saccharide **6** répondant à la formule générale (IVbis), dans laquelle P¹ représente Bn, A représente O, L représente CH₂-CH₂ et L₁ représente Br, est illustré ci-dessous :

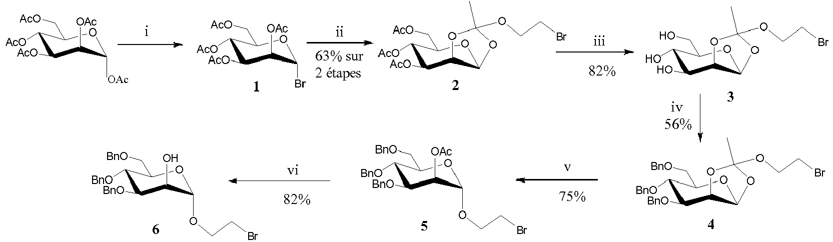

Le composé de départ est le α-D-mannose pentaacétylé sur lequel est introduit un brome en position anomère à l'aide d'acide bromhydrique à 33% dans l'acide acétique pour former le composé **1.** L'intermédiaire **1** est ensuite mis en réaction avec du 2-bromoéthanol et de la 2,6-lutidine pour former l'orthoester **2** avec un rendement de 63% sur deux étapes. Les acétates présents sur les positions 3, 4 et 6 de l'orthoester **2** sont ensuite saponifiés pour conduire à l'intermédiaire **3** avec un rendement de 82%. Cette étape de déprotection est totale et ne nécessite pas de purification. Ces mêmes positions 3, 4 et 6 sont ensuite benzylées par l'action du bromure de benzyle pour former le composé **4** avec un rendement de 56%. L'orthoester **4** est ensuite ouvert en présence de BF₃.Et₂O et de 2-bromoéthanol pour former l'intermédiaire clé **5** avec un rendement de 75%. Le composé **5** est ainsi fonctionnalisé en position anomère et porte un groupement protecteur en position 2 orthogonal aux groupements des positions 3, 4 et 6. On peut ainsi déprotéger sélectivement cette position 2 à l'aide d'une solution de soude pour obtenir l'intermédiaire **6** avec un rendement de 82%.

L'accepteur de saccharide **6** est fonctionnalisé en position anomère : le synthon **6** sera donc commun quel que soit le disaccharide de formule (II) souhaité.

Conditions et réactifs : (i) HBr 33%, AcOH, TA, 1h ; (ii) 2-bromoéthanol, 2-6-lutidine, DCM, 40°C, 3h ; (iii) NaOH 1N, THF, TA, 16h ; (iv) BnBr, NaH, DMF, TA, 21h ; (v) 2-bromoéthanol, BF₃.Et₂O, TA, 1h30 ; (vi) NaOH 1N, THF, TA 21h.

### Partie expérimentale

### Préparation du 1-bromo-2,3,4,6-tétra-O-acétyl-α-D-mannopyranose 1:

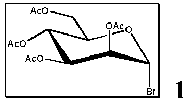

10g d'α-D-mannose pentaacétate sont dissous dans 20 mL d'une solution d'acide bromhydrique à 33% dans l'acide acétique. La solution jaune est agitée 1 h à température ambiante. Le mélange réactionnel est refroidi à 0°C, dilué par 30 mL de CH₂Cl₂ et neutralisé à l'aide d'une solution saturée de NaHCO₃. La phase aqueuse est extraite avec de CH₂Cl₂ (5x100 mL). Les phases organiques sont combinées, séchées sur MgSO₄ et concentrées pour conduire au composé **1** (11,9 g) qui est utilisé pour l'étape suivante sans purification.

Formule chimique **1** : C₁₄H₁₉BrO₉

Masse exacte : 410,02 g.mol⁻¹
R_{f} : 0,76 [AcOEt/Cyclo (1:1)]

### Préparation du 1,2-O-(1-(2-bromoéthoxy)éthylidène)-3,4,6-tri-O-acétyl-α-D-mannopyranose 2 :

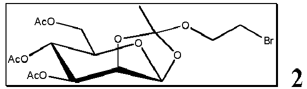

11,08 g (1 éq ; 26,9 mmol) du composé 1 sont dissous dans 9,5 mL de CH₂Cl₂ et 9,4 mL (3 éq ; 80,7 mmol) de 2,6-lutidine. 4,8 mL (2,5 éq ; 67,25 mmol) de 2-bromoéthanol sont ajoutés. Le milieu réactionnel de couleur orange est chauffé à 40°C et agité pendant 4 h. Un précipité blanc est formé. La solution est refroidie à température ambiante et 12 mL d'Et₂O sont ajoutés. Le précipité est filtré. Le filtrat est dilué avec 30 mL de CH₂Cl₂ et lavé successivement avec 30 mL d'eau, 30 mL d'une solution saturée de NaHCO₃ et 30 mL de saumure. Les phases aqueuses combinées sont extraites avec 50 mL de CH₂Cl₂. Les phases organiques sont combinées puis séchées sur MgSO₄ et concentrées. Le résidu obtenu est dissous dans 20 mL de CH₂Cl₂ et 25 mL d'éthanol sont ajoutés. La solution est refroidie à -30°C pour conduire au composé **2** sous la forme de cristaux blancs avec un rendement de 51% (6,268 g; 13,8 mmol) sur deux étapes.

Formule chimique **2** : C₁₆H₂₃BrO₁₀

Masse exacte : 454,05 g.mol⁻¹
R_{f} : 0,49 [Cyclo/AcOEt (1:1)]
SM, ESI⁺ m/z : 477 [M+Na]⁺

### Préparation du 1,2-O-(1-(2-bromoéthoxy)éthylidène-α-D-mannopyranose 3:

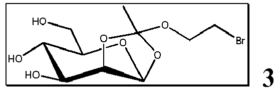

4,79 g (1 éq ; 10,6 mmol) du composé **2** sont dissous dans 22 mL de THF. 43 mL d'une solution aqueuse de NaOH 1N sont ajoutés. La solution devient trouble. 2 mL de méthanol sont ajoutés et la solution devient limpide. Le mélange réactionnel est agité à température ambiante pendant 16 h. 250 mL d'acétate d'éthyle sont ajoutés et la phase aqueuse est extraite avec 3x200 mL d'acétate d'éthyle. Les phases organiques sont combinées puis séchées sur MgSO₄ et concentrées. Le résidu obtenu est purifié par chromatographie flash automatisée sur gel de silice (CH₂Cl₂/MeOH : de 0 à 5% en 15 min; 5% pendant 10 min; de 5 à 10% en 15 min) pour conduire au composé 3 sous forme d'une huile incolore avec un rendement de 82% (2,86 g ; 8,69 mmol).

Formule chimique **3** : C₁₀H₁₇BrO₇

Masse exacte : 328,02 g.mol⁻¹
R_{f} : 0,36 [CH₂Cl₂/MeOH (9:1)]
SM, ESI⁻m/z : 373 [M-H+HCOOH]⁻

### Préparation du 1,2-O-(1-(2-bromoéthoxy)éthylidène-3,4,6-tri-O-benzyl-α-D-mannopyranose 4

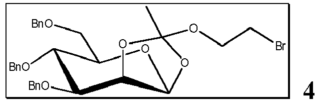

2,86 g (1 éq ; 8,69 mmol) du composé **3** sont dissous dans 40 mL de DMF. 4,2 mL (4 éq ; 34,76 mmol) de bromure de benzyle sont ajoutés. Le mélange réactionnel est refroidi à 0°C et 874 mg (3 éq ; 26 mmol) de NaH 60% dispersion dans l'huile sont ajoutés. Après 17h30 d'agitation à température ambiante, il reste du produit partiellement benzylé. L'ajout d'un équivalent de bromure de benzyle et de NaH ne fait pas évoluer la réaction. 50 mL d'Et₂O et 40 mL d'eau sont ajoutés. La phase aqueuse est extraite avec 2x100 mL d'Et₂O. Les phases organiques sont combinées, lavées avec 5x80 mL d'eau puis séchées sur MgSO₄ et concentrées. Le résidu obtenu est purifié par chromatographie flash automatisée sur gel de silice (Cyclohexane/Et₂O : de 0 à 10% en 15 min; 10% pendant 10 min; de 10 à 20% en 15 min; 20% pendant 50 min; de 20 à 40% en 30 min) pour conduire au composé **4** sous forme de solide blanc avec un rendement de 56% (2,92 g ; 4,87 mmol).

Formule chimique **4** : C₃₁H₃₅BrO₇

Masse exacte : 598,16 g.mol⁻¹
R_{f} : 0,43 [Cyclo/Et₂O (1: 1)]
SM, ESI⁺ m/z : 621 [M+Na]⁺

### Préparation du 2-O-(3-acétyl-3,4,6-tri-O-(3-benzyl-α-D-mannopyranoside de 2-bromoéthyle 5

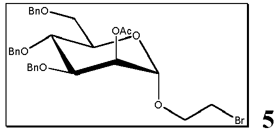

1g (1 éq ; 1,67 mmol) du composé **4** est dissous dans 20mL de CH₂Cl₂. 236 µL (2 éq ; 3,34 mmol) de 2-bromoéthanol sont ajoutés et le mélange réactionnel est agité à température ambiante pendant 10 min. 211µL (1 éq ; 1,67 mmol) de BF₃.Et₂O. Le mélange réactionnel est agité à température ambiante pendant 16 h. La solution est devenue jaune pâle. La réaction est diluée avec 50 mL de solution saturée de NaHCO3. La phase aqueuse est extraite avec 3x50 mL de CH₂Cl₂. Les phases organiques sont combinées, lavées avec 100 mL de saumure puis séchées sur MgSO₄ et concentrées. Le résidu obtenu est purifié par chromatographie flash automatisée sur gel de silice (Cyclohexane/Et₂O: 0 à 30% en 30 min ; 30% pendant 20 min) pour conduire au composé **5** sous forme d'une huile incolore avec un rendement de 75% (750 mg ; 1,25 mmol).

Formule chimique **5** : C₃₁H₃₅BrO₇

Masse exacte : 598,16 g.mol⁻¹
R_{f} : 0,45 [Cyclo/Et₂O (1: 1)]
SM, ESI⁺ m/z : 621 [M+Na]⁺

### Préparation du 3,4,6-tri-O-benzyl-α-D-mannopyranoside de 2-bromoéthyle 6 :

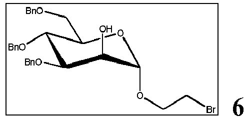

750 mg (1 éq ; 1,25 mmol) du composé **5** sont dissous dans 1,6 mL de THF. 2,5 mL (2 éq ; 2,5 mmol) d'une solution de NaOH 1N sont ajoutés. La solution devient trouble. La solution est agitée à température ambiante pendant 21 h. La solution est neutralisée avec une solution d'HCl 1N. 40 mL de CH₂Cl₂ sont ajoutés. La phase aqueuse est extraite avec 3x40 mL de CH₂Cl₂. Les phases organiques sont combinées puis séchées sur MgSO₄ et concentrées. Le résidu obtenu est purifié par chromatographie flash automatisée sur gel de silice (Cyclohexane/Et₂O: de 0 à 20% en 15 min; 20% pendant 10 min; de 20 à 30% en 15 min ; 30% pendant 15 min) pour conduire au composé **6** sous forme d'une huile incolore avec un rendement de 82% (575 mg ; 1,03 mmol).

Formule chimique **6** : C₂₉H₃₃BrO₆

Masse exacte : 556,16 g.mol⁻¹
R_{f} : 0,29 [Cyclo/Et₂O (7:3)]
SM, ESI⁺ m/z : 579 [M+Na]⁺

### b) Préparation d'un donneur de saccharide 8 : le trichloroacétimidate de 2,3,4,6-tétra-O-acétyl-α-D-mannopyranose

Le procédé de préparation d'un composé donneur de saccharide **8** répondant à la formule générale (V), dans laquelle P¹ représente Ac, est illustré ci-dessous :

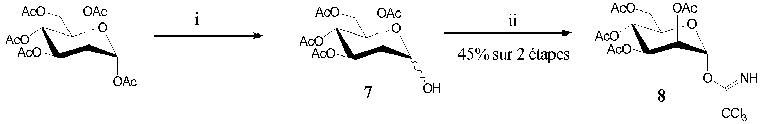

Le composé de départ est le α-D-mannose pentaacétylé qui est déprotégé en position anomère à l'aide de morpholine pour conduire au composé **7** sans purification. Le trichloroacétimidate **8** est ensuite formé par réaction du composé **7** avec du trichloroacétonitrile en présence de DBU.

Conditions et réactifs : (i) morpholine, DCM, reflux, 3h30 ; (ii) Cl₃CCN, DBU, DCM, TA, 4 h.

### Partie expérimentale

### Préparation du 2,3,4,6-tétra-O-acétyl-D-mannose 7:

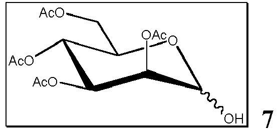

3 g (1 éq ; 7,69 mmol) d'α-D-mannose pentaacétate sont dissous dans 18 ml de CH₂Cl₂. 2,7 mL (4 éq ; 30,76 mmol) de morpholine sont ajoutés. Le mélange réactionnel est chauffé à reflux pendant 3h30 puis refroidi à température ambiante. La solution est neutralisée avec une solution d' HCl 1N. La phase organique est lavée avec 3x10 mL d'eau, séchée sur MgSO₄ et concentrée pour conduire au produit 7 sous forme d'une huile jaune directement utilisée pour l'étape suivante.

Formule chimique 7 : C₁₄H₂₀O₁₀

Masse exacte : 348,30 g.mol⁻¹
R_{f} : 0,38 [Cyclo/AcOEt (1:1)]
SM, ESI⁻ m/z : 393 [M-H+HCOOH]⁻

### Préparation du trichloroacétimidate de 2,3,4,6-tétra-O-acétyl-α-D-mannopyranose 8:

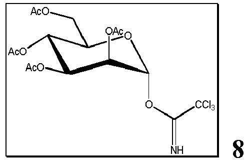

1,34g (1 éq ; 3,85 mmol) du composé 7 sont dissous dans 8,6 mL de CH₂Cl₂. La solution de couleur jaune est refroidie à 0°C. 2,7 mL (7 éq ; 26,95 mmol) de trichloroacétonitrile et 575 µL de DBU (1,8-diazabicyclo[5.5.0]undec-7-ène) sont ajoutés. La solution devient marron. Le milieu réactionnel est agité 4 h à température ambiante puis concentré. Le résidu obtenu est purifié par chromatographie flash automatisée sur gel de silice (Cyclohexane/Et₂O : 50% pendant 25 min) pour conduire au produit **8** sous forme d'huile jaune pâle avec un rendement de 45% (860 mg ; 1,75 mmol). Le produit relativement instable est utilisé rapidement.

Formule chimique **8** : C₁₆H₂₀Cl₃NO₁₀

Masse exacte : 492,69 g.mol⁻¹
R_{f} : 0,4 [Cyclo/Et₂O (3:7)]

### c) Préparation des disaccharides 9, 10, 11 et 12

Le procédé de préparation de disaccharides **9, 10, 11** et **12** répondant à la formule (II) est illustré ci-dessous :

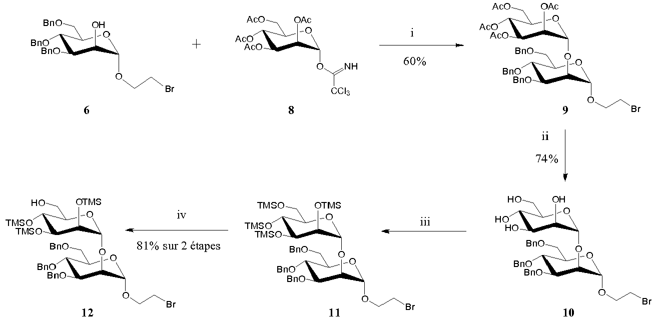

La réaction de glycosylation entre l'accepteur de formule IV ou IVbis, en particulier le saccharide **6,** et le donneur de formule V, en particulier le saccharide **8,** permet l'obtention du composé de formule (II), en particulier le disaccharide **12,** qui occupe une position centrale dans la stratégie de synthèse de l'invention. En effet, le composé de formule (II) permet de diverger vers l'ensemble des composés de formule (I) de l'invention. Les analogues isostères du M6P de l'invention (I) sont en effet accessibles à partir du composé de formule (II).

Le procédé de synthèse de l'invention confère de la souplesse dans la production des analogues disaccharidiques de formule (I) de l'invention mais également aux analogues tri- ou tétra-saccharidiques de formule (I) de l'invention.

Les composés respectifs « accepteur de saccharide » **6** et « donneur de saccharide » **8** tels que préparés ci-dessus sont mis en réaction en présence de triflate de triméthylsilyle pour former le disaccharide **9** avec un rendement de 60%. Les groupements acétates sont ensuite saponifiés pour conduire au composé **10** avec un rendement de 74%. Les alcools ainsi déprotégés sont ensuite triméthylsilylés par action du chlorure de triméthylsilyle pour former l'intermédiaire **11.** La position 6 est ensuite désilylée sélectivement en présence de carbonate de potassium dans le méthanol pour conduire au disaccharide **12** avec un rendement de 81% sur deux étapes.

Conditions et réactifs : (i) TMSOTf, DCM, -30°C, 30min ; (ii) NaOH 1N, THF, TA, 18h ; (iii) TMSCl, NEt₃, DCM, TA, 18h; (iv) K₂CO₃, MeOH, TA, 1h45.

### Partie expérimentale

### Préparation du 2,3,4,6-tétra-O-acétyl-α-D-mannopyranosyl-(1→2)-3,4,6-tri-O-benzyl-α-D-mannopyranoside de bromoéthyle 9 :

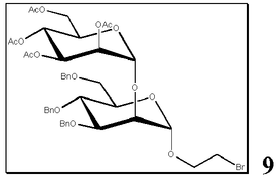

430 mg (1éq ; 0,77 mmol) du composé **6** et 860 mg (2,27 éq ; 1,75 mmol) du composé **8** sont dissous dans 15 mL de CH₂Cl₂ en présence de 7 g de tamis moléculaire 4Â précédemment activé. Le milieu réactionnel est agité 30 min à température ambiante puis refroidi à -30°C. 167 µL (1,2 éq ; 0,924 mmol) de TMSOTf sont ajoutés goutte à goutte. Le mélange réactionnel est agité à -30°C pendant 1h15. Le milieu réactionnel est ensuite neutralisé avec 3,5 mL de pyridine. Après filtration sur célite, le filtrat est concentré puis co-évaporé avec du toluène. Le résidu (solide jaune pâle) obtenu est purifié par chromatographie flash automatisée sur gel de silice (Cyclohexane/Et₂O: 30% pendant 110 min) pour conduire au produit **9** sous la forme d'une huile incolore avec un rendement de 64% (441mg ; 0,497 mmol).

Formule chimique **9** : C₄₃H₅₁BrO₁₅

Masse exacte : 886,24 g.mol⁻¹
R_{f} : 0,56 [Cyclo/Et₂O (2:8)]
SM, ESI⁺ m/z : 909 [M+Na]⁺

### Préparation du α-D-mannopyranosyl-(1→2)-3,4,6-tri-O-benzyl-α-D-mannopyranoside de bromoéthyle 10 :

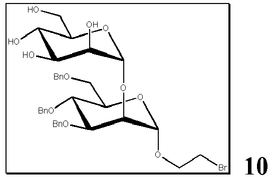

5,84 g (1 éq, 6,58 mmol) du composé **9** sont dissous dans 16 mL de THF. 33 mL (5 éq, 32,9 mmol) d'une solution de soude 1 M sont ajoutés. La solution devient trouble. 1,5 mL de méthanol sont ajoutés. La solution est agitée à température ambiante pendant 18h puis neutralisée avec une solution de HCl 1M et concentrée. Le résidu obtenu est purifié par chromatographie flash sur gel de silice (DCM/MeOH : 0% (500 mL); 5% (500 mL); 7% (500 mL); 10% (1 L)) pour conduire au composé **10** sous la forme d'une mousse blanche avec un rendement de 74% (3,5 g, 4,86 mmol).

Formule chimique **10** : C₃₅H₄₃BrO₁₁

Masse molaire : 718,2 g.mol⁻¹
R_{f} : 0,41 [DCM/MeOH (9:1)]

### Préparation du 2,3,4,6-tétra-O-triméthylsilyl-α-D-mannopyranosyl-(1→2)-3,4,6-tri-O-benzyl-α-D-mannopyranoside de bromoéthyle 11 :

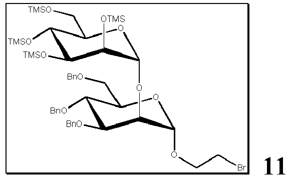

3,4 g (1 éq, 4,72 mmol) du composé **10** sont dissous dans 24 mL de DCM fraîchement distillé et 19 mL (30éq ; 141,6 mmol) de triéthylamine. La solution est refroidie à 0°C et 4,8 mL (8 éq ; 37,8 mmol) de chlorure de triméthylsilyle sont ajoutés goutte à goutte. Après 18 h d'agitation à température ambiante, il reste un peu de produit de départ. 1,2 mL (2 éq ; 9,44 mmol) de chlorure de triméthylsilyle sont ajoutés goutte à goutte. Après 1h30 d'agitation à température ambiante, il n'y a pas d'évolution. La solution est concentrée. Le résidu rose est dissous dans du cyclohexane et filtré sur célite. Le filtrat est concentré pour obtenir le composé **11** sous la forme d'une huile marron clair directement utilisée pour l'étape suivante.

Formule chimique **11** : C₄₇H₇₅BrO₁₁Si₄

Masse molaire : 1008,34 g.mol⁻¹
R_{f} : 0,78 [Cyclo/Et₂O (7:3)]

### Préparation du 2,3,4-tri-O-triméthylsilyl-α-D-mannopyranosyl-(1→2)-3,4,6-tri-O-benzyl-α-D-mannopyranoside de bromoéthyle 12 :

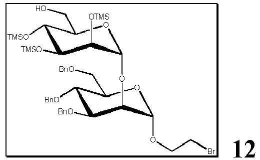

4,58 g (4,72 mmol ; 1 éq) du composé **11** sont dissous dans 65 mL de méthanol fraîchement distillé. Une solution de 6,5 mg (0,01 éq ; 0,0472 mmol) de K₂CO₃ (concentration finale = 0,63 mM) dans 10 mL de méthanol est ajoutée goutte à goutte. Le milieu réactionnel est agité 1h45 à température ambiante puis dilué avec 200 mL de CH₂Cl₂ et lavé avec 125 mL de saumure. La phase aqueuse est extraite avec 260 mL de CH₂Cl₂. Les phases organiques sont combinées, séchées sur MgSO₄ et concentrées. Le résidu obtenu (huile marron clair) est purifié par chromatographie sur gel de silice (Cyclohexane/Et₂O : 30% (700 mL) ; 40% (500 mL) ; 50% (500 mL) ; 70% (500 mL)) pour conduire au composé **12** (3,58 g; 3,82 mmol) sous la forme d'un liquide incolore avec un rendement de 81% sur 2 étapes. Formule chimique **12** : C₄₄H₆₇BrO₁₁Si₃ Masse molaire : 936,16g.mol⁻¹ R_{f} : 0,45 [Cyclo/Et₂O (1: 1)] SM, ESI⁺ m/z : 959 [M+Na]⁺

### 2) Préparation de disaccharides 14, 15, 16, 17, 18 et 19 répondant à la formule (I) dans laquelle X représente le groupement phosphonate

Le procédé de préparation de disaccharides **14, 15, 16, 17, 18** et **19** répondant à la formule (**I**) dans laquelle X représente le groupement phosphonate est illustré ci-dessous :

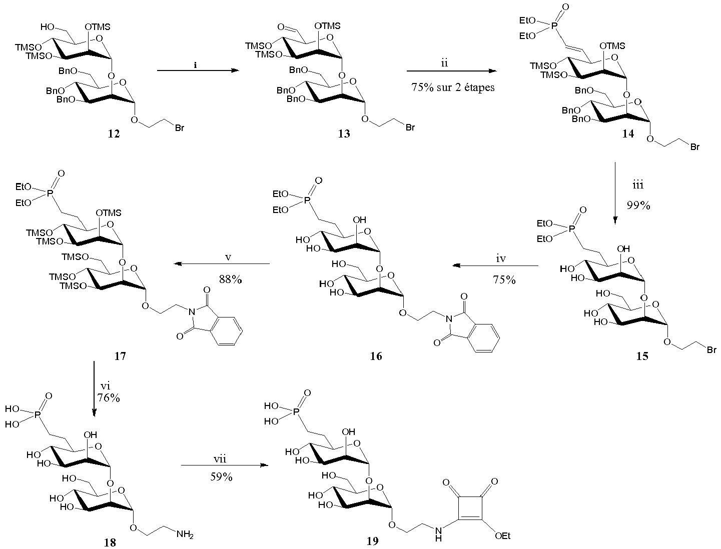

L'alcool **12** obtenu tel que décrit précédemment est oxydé en aldéhyde à l'aide du périodinane de Dess-Martin pour former le composé **13.** Cet intermédiaire clé va permettre l'accès aux différents disaccharides de formule (I).

Pour synthétiser les disaccharides « phosphonate » de formule (I), l'aldéhyde **13** est mis en réaction avec le méthylènediphosphonate de tétraéthyle, préalablement déprotoné par de l'hydrure de sodium, pour former le composé **14** avec un rendement de 75% sur deux étapes. Lors d'une étape d'hydrogénation catalytique, en présence de palladium sur charbon et pour laquelle l'hydrogène est généré in situ par ajout progressif de triéthylsilane, la double liaison ainsi que les trois benzyles sont réduits et les groupements triméthylsilyles sont hydrolysés. On obtient ainsi le composé **15,** sans purification, avec un rendement quantitatif. L'atome de brome est ensuite substitué par du phtalimide de potassium pour former l'intermédiaire **16** avec un rendement de 75%. Les fonctions alcools sont ensuite protégées par du chlorure de triméthylsilyle pour conduire au composé **17** avec un rendement de 88%. L'intermédiaire **17** ainsi obtenu, sans purification est ensuite mis en réaction avec du chlorure de triméthylsilyle et de l'iodure de sodium pour former le phosphonate bis(triméthylsilylé) par une réaction de type Rabinowitz. Cet intermédiaire est ensuite converti en acide phosphonique par action de l'hydrazine monohydratée dans le méthanol qui va aussi déplacer les groupements triméthylsilyles présents sur les alcools secondaires du sucre et réagir avec le phtalimide afin de démasquer la fonction amine. On obtient ainsi, en une seule étape, le composé **18** déprotégé sur la partie phosphonate, sur les alcools des deux sucres et portant la fonction amine en position anomère avec un rendement de 76% sur deux étapes. Le composé **18** est ensuite mis en réaction avec le squarate de diéthyle pour former le produit **19** avec un rendement de 59%.

Conditions et réactifs : (i) Dess-Martin periodinane, DCM, TA, 4h (ii) méthylènediphosphonate de tétraéthyle, NaH, THF, TA, 45min ; (iii) Pd/C, Et₃SiH, MeOH ; (iv) Phtalimide de potassium, DMF, 60°C, 40h ; (v) TMSCl, NEt₃, DCM, DMF, TA, 24h ; (vi)-a TMSCl, NaI, ACN, 35°C, 1h ; (vi)-b N₂H₄.H₂O, MeOH, TA, 1h30 ; (vii) squarate de diéthyle, EtOH/H₂O, NEt₃.

### Partie expérimentale

### Préparation du 2,3,4-tri-O-triméthylsilyl-α-D-manno-hexodialdopyranosyl.(1→2)-3,4,6-tri-O-benzyl-α-D-mannopyranoside de bromoéthyle 13:

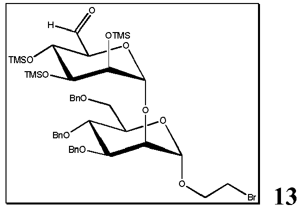

1 g (1 éq ; 1,07 mmol) du composé **12** est dissous dans 24 mL de DCM sur tamis moléculaire. 5,4 mL (1,5 éq ; 1,6 mmol) d'une solution de périodinane de Dess-Martin à 0,3 M dans le dichlorométhane sont ajoutés goutte à goutte. Le milieu réactionnel est agité à température ambiante pendant 4 h puis dilué avec 120 mL d'Et₂O. 25 mL d'une solution saturée de NaHCO₃ et 3,9 g de Na₂S₂O₃ sont ajoutés. La solution est agitée à température ambiante pendant 5 min. La phase aqueuse est extraite avec 3x125 mL d'Et₂O. Les phases organiques sont combinées, séchées sur MgSO₄ et concentrées pour conduire au composé **13** utilisé directement pour l'étape suivante.

Formule chimique **13** : C₄₄H₆₅BrO₁₁Si₃

Masse molaire : 934,14g.mol⁻¹
R_{f} : 0,77 [DCM/Et₂O (95:5)]

### Préparation du 2,3,4-tri-O-triméthylsilyl-6,7-didésoxy-7-diéthoxyphosphinyl-αD-manno-hept-6-énopyranosyl-(1→2)-3,4,6-tri-O-benzyl-α-D-mannopyranoside de bromoéthyle 14 :

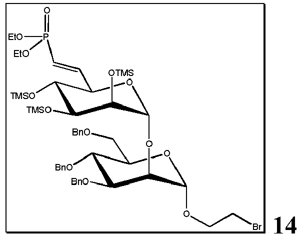

90 mg (2,5 éq ; 2,67 mmol) de NaH 60% dispersion dans l'huile sont dissous dans 16 mL de THF. 530µL (2 éq ; 2,14 mmol) de méthylènediphosphonate de tétraéthyle sont ajoutés goutte à goutte. Le mélange réactionnel est agité 45 min à température ambiante puis ajouté à 998 mg (1 éq ; 1,07 mmol) du composé **13** dissous dans 8 mL de THF. La solution se colore en jaune puis devient marron. Le milieu réactionnel est agité à température ambiante pendant 45min puis dilué avec 200mL de CH₂Cl₂ et lavé avec 2x40 mL de saumure. La phase aqueuse est extraite avec 3x130 mL de CH₂Cl₂. Les phases organiques sont combinées, séchées sur MgSO₄ et concentrées. Le résidu obtenu (huile marron) est purifié par chromatographie flash automatisée sur gel de silice (DCM/Et₂O : 0% à 5% en 15 min; 5% pendant 10 min; de 5% à 10% en 15 min; 10% pendant 10 min ; de 10 à 13% en 15 min) pour conduire au composé **14** sous la forme d'une huile jaune clair avec un rendement de 76% (864 mg ; 0,81 mmol) sur deux étapes.

Formule chimique **14** : C₄₉H₇₆BrO₁₃S₁₃

Masse molaire : 1068,25 g.mol⁻¹
R_{f} : 0,26 [DCM/Et₂O (95:5)]

### Préparation du 6-désoxy-6-diéthoxyphosphinylméthylène-α-D-mannopyranosyl-(1→2)-α-D-mannopyranoside de bromoéthyle 15 :

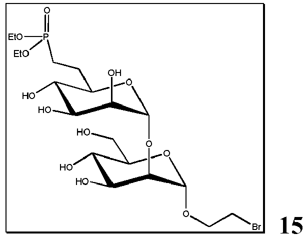

864 mg (0,81 mmol) du composé **14** sont dissous dans 8,2 mL de méthanol. 130 mg (15% massique) de palladium sur charbon sont ajoutés. 1,3 mL (8,1 mmol ; 10 éq) de triéthylsilane sont ajoutés goutte à goutte pendant 1h20. La suspension est agitée à température ambiante pendant 30 min puis filtrée sur célite. Le filtrat est évaporé pour conduire au composé **15** avec un rendement de 99% (468 mg ; 8,0 mmol).

Formule chimique **15** : C₁₉H₃₆BrO₁₃P

Masse exacte : 582,2 g.mol⁻¹
R_{f} : 0,23 [DCM/MeOH (85:15)]
SM, ESI⁺ m/z : 583,1 [M+H]⁺

### Préparation du 6-désoxy-6-diéthoxyphosphinylméthylène-α-D-mannopyranosyl-(1→2)-α-D-mannopyranoside de phtalimidoéthyle 16:

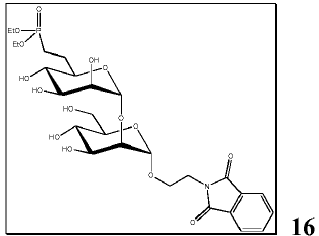

220 mg (0,38 mmol) du composé **15** sont dissous dans 1,8 mL de DMF sur tamis moléculaire. 119 mg (0,64 mmol ; 1,7 éq) de phtalimide de potassium sont ajoutés. La suspension est agitée pendant 40 h à 60°C puis refroidie à température ambiante et concentrée. Le résidu obtenu est purifié par chromatographie sur gel de silice (AcOEt/MeOH : 75/25) pour conduire au composé **16** sous la forme d'un solide incolore avec un rendement de 75% (184 mg ; 0,28 mmol).

Formule chimique **16 :** C₂₇H₄₀NO₁₅P

Masse molaire : 649,58 g.mol⁻¹
R_{f} : 0,31 [AcOEt/MeOH (75:25)]
SM, ESI⁺ m/z : 650,3 [M+H]⁺

### Préparation du 2,3,4-tri-O-triméthylsilyl-6-désoxy-6-diéthoxyphosphinylméthylène-α-D-mannopyranosyl-(1→2)-3,4,6-tri-O-triméthylsilyl-α-D-mannopyranoside de phtalimidoéthyle 17:

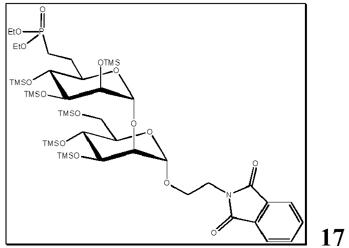

184 mg (0,28 mmol) du composé **16** sont dissous dans 817 µL de DCM fraîchement distillé et 1,15 mL (8,5 mmol ; 30 éq) de triéthylamine. Le produit de départ n'est pas complètement soluble. 431 µL (3,4 mmol ; 12 éq) de chlorure de triméthylsilyle sont ajoutés goutte à goutte. La solution est agitée à température ambiante pendant 15 h. Le produit de départ n'est toujours pas complètement solubilisé et la solution est de couleur blanche. 0,5 mL de DMF sur tamis moléculaire sont ajoutés, le produit de départ est alors soluble. La solution est agitée à température ambiante pendant 24 h puis évaporée. Au cours de la réaction, la solution devient marron. Le résidu marron est dissous dans du cyclohexane puis filtré sur célite. Le filtrat est concentré pour conduire au composé **17** avec un rendement de 88% (268 mg ; 0,25 mmol) directement utilisé pour l'étape suivante.

Formule chimique **17 :** C₄₅H₈₈NO₁₅PSi₆

Masse molaire : 1082,66g.mol⁻¹
R_{f} : 0,49 [DCM/Et₂O (9:1)]

### Préparation du 6-désoxy-6-dihydroxyphosphinylméthylène-α-D-mannopyranosyl-(1→2)-D-mannopyranoside d'aminoéthyle (M6Pn-α(1,2)-Man-Ethyl-NH₂) 18:

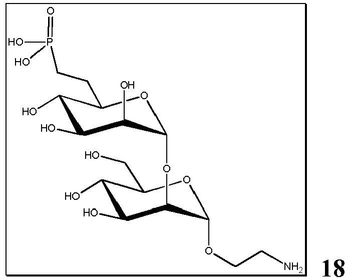

269 mg (0,25 mmol) du composé **17** et 260 mg (1,73 mmol ; 7 éq) d'iodure de sodium sont dissous dans 7,3 mL d'acétonitrile fraîchement distillé. 219 µL (1,73 mmol ; 7 éq) de chlorure de triméthylsilyle sont ajoutés goutte à goutte. La solution est agitée à 35°C pendant 1h15. Pendant la réaction un précipité se forme. Le surnageant est canulé dans un ballon et évaporé à sec. 132 µL (2,72 mmol ; 11 éq) d'hydrazine monohydratée sont dilués dans 3,4 mL de méthanol sur tamis moléculaire et ajoutés au résidu obtenu précédemment. Un précipité blanc est formé. La suspension est agitée à température ambiante pendant 1h30. Le précipité est dissous dans l'eau et évaporé. Le résidu obtenu est purifié par chromatographie sur gel de silice (isopropanol/ammoniaque/eau : 5/3/2) pour conduire au composé **18** sous la forme d'un solide blanc avec un rendement de 76% (67 mg ; 0,14 mmol).

Formule chimique **18 :** C₁₅H₃₀NO₁₃P

Masse molaire : 463,37 g.mol⁻¹
R_{f} : 0,49 [DCM/Et₂O (9:1)]
SMHR : masse calculée : 464,1533 ; masse trouvée : 464,1530

### Préparation du 6,7-didésoxy-7-dihydroxyphosphinyl-α-D-manno-heptopyranosyl-(1→2)-α-D-mannopyranoside de (4-éthoxy-2,3-dioxocyclobut-1-ényl)aminoéthyle (M6Pn-α(1,2)-Man-EthylSq) 19:

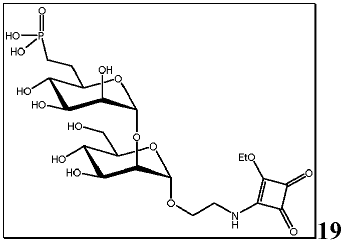

Le composé **18** (32mg ; 1 éq ; 0,07mmol) est dissous dans un mélange éthanol/eau (1:1) (620µL). Le squarate de diéthyle (10,3µL ; 1 éq ; 0,07 mmol) et la triéthylamine (9,7µL ; 1 éq ; 0,07mmol) sont ajoutés. La réaction est agitée à température ambiante pendant 20h puis le solvant est évaporé. Le résidu est précipité dans un mélange AcOEt/MeOH (7:3). Après centrifugation, le précipité est rincé 5 fois avec de l'acétate d'éthyle pour conduire au composé **19** avec un rendement de 59% (24mg ; 0,041 mmol).

Formule chimique **19 :** C₂₁H₃₄NO₁₆P

Masse molaire : 587,47g.mol⁻¹
R_{f} : 0,67 [MeOH/H₂O (9:1)]
SM, ESI⁻ m/z : 586 [M-H]⁻

### 3) Préparation de disaccharides 20, 21, 22, 23, 24, et 25 répondant à la formule (I) dans laquelle X représente le groupement carboxylate

Le procédé de préparation de disaccharides **20, 21, 22, 23, 24** et **25** répondant à la formule (I) dans laquelle X représente le groupement carboxylate est illustré ci-dessous :

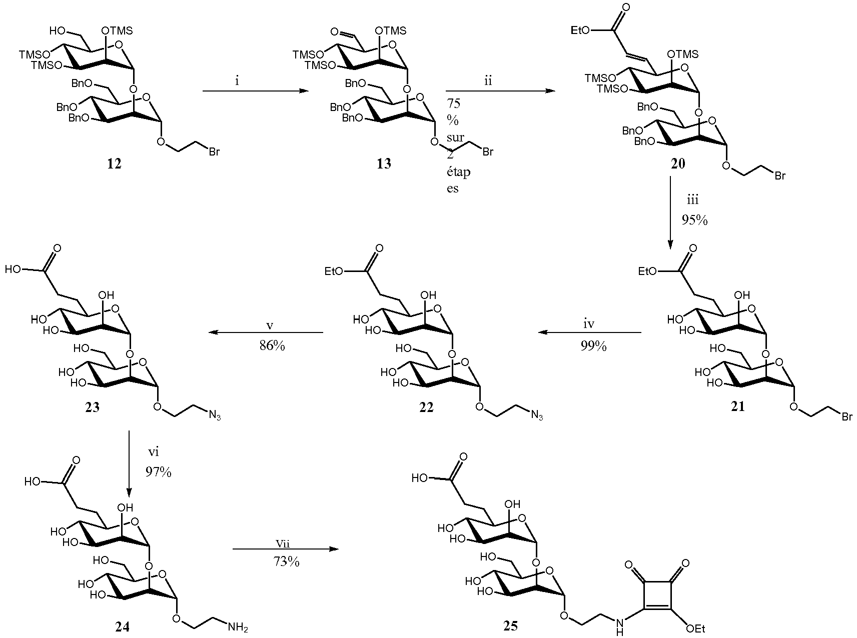

Le début de la synthèse des disaccharides « carboxylate » de formule (I) est identique à celle des disaccharides « phosphonate », à savoir l'aldéhyde **13** est obtenu à partir de l'alcool **12** de la même manière que décrit ci-dessus.

L'aldéhyde **13** est ensuite mis en réaction avec du phosphonoacétate de triéthyle préalablement déprotoné à l'aide d'hydrure de sodium pour former le composé **20** avec un rendement de 75% sur deux étapes. En une seule étape d'hydrogénation, la double liaison ainsi que les 3 groupements benzyles sont réduits. Lors de cette réaction, les groupements triméthylsilyles sont aussi hydrolysés. Cette réaction est effectuée à l'aide de palladium sur charbon à 10% et l'hydrogène est généré in situ par ajout progressif de triéthylsilane pour former le composé **21** avec un rendement de 95%. Cette étape d'hydrogénation est totale et ne nécessite pas de purification pour obtenir le composé **21** pur. L'atome de brome est ensuite substitué par de l'azoture de sodium pour conduire au composé **22** avec un rendement de 99%. La fonction ester est alors saponifiée pour former le composé **23** avec un rendement de 86%. Enfin la fonction azide est réduite lors d'une réaction d'hydrogénation catalytique à l'aide de palladium sur charbon et de triéthylsilane pour former le composé **24** avec un rendement de 97%. Enfin, le composé **24** est mis en réaction avec du squarate de diéthyle pour conduire au composé **25** avec un rendement de 73%.

Conditions et réactifs : (i) Dess-Martin periodinane, DCM, TA, 4h; (ii) phosphonoacetate de triéthyle, NaH, THF, TA, 14h ; (iii) Pd/C, Et₃SiH, MeOH, TA ; (iv) NaN₃, DMF, TA, 5j ; (v) NaOH 1N, TA, 20h ; (vi) Pd/C, Et₃SiH, MeOH/H₂O ; (vii) squarate de diéthyle, EtOH/H₂O, NEt₃, TA, 2h30.

### Partie expérimentale

### Préparation du 2,3,4-tri-O-triméthylsilyl-6,7-didésoxy-7-éthoxycarbonyl-α-D-mannohept-6-énopyranosyl-(1→2)-3,4,6-tri-O-benzyl-α-D-mannopyrannoside de bromoéthyle 20 :

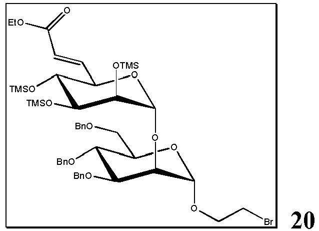

144 mg (4 éq ; 4,28 mmol) de NaH 60% dispersion dans l'huile sont dissous dans 10mL de THF. 637 µL (3 éq ; 3,21 mmol) de phosphonoacétate de triéthyle sont ajoutés goutte à goutte. Le mélange réactionnel est agité 45 min à température ambiante puis ajouté à 1,065 g (1 éq ; 1,07 mmol) du composé **13** dissous dans 20 mL de THF. La solution se colore en orange. Le milieu réactionnel est agité à température ambiante pendant 14h30 puis dilué avec 175 mL de CH₂Cl₂ et lavé avec 4x50 mL de saumure. La phase aqueuse est extraite avec 125 mL de CH₂Cl₂. Les phases organiques sont combinées, séchées sur MgSO₄ et concentrées. Le résidu obtenu (huile orange) est purifié par chromatographie flash automatisée sur gel de silice (Cyclohexane/Et₂O : 20% (900 mL)) pour conduire au composé **20** sous la forme d'une huile incolore avec un rendement de 75% (802 mg ; 0,80 mmol) sur deux étapes.

Formule chimique **20** : C₄₈H₇₁BrO₁₂Si₃

Masse molaire : 1004,23 g.mol⁻¹
R_{f} : 0,39 [Cyclo/Et₂O (8:2)]

### Préparation du 6,7-didésoxy-7-éthoxycarbonyl-α-D-manno-heptopyranosyl-(1 →2)-α-D-mannopyranoside de bromoéthyle 21 :

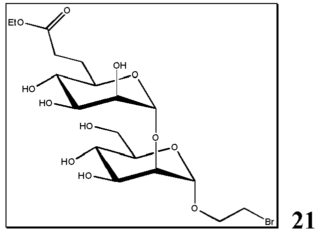

795 mg (1 éq ; 0,79 mmol) du composé **20** sont dissous dans 8 mL de méthanol. 160 mg (20% massique) de palladium sur charbon à 10% sont ajoutés. 1,3 mL (10 éq ; 7,9 mmol) de triéthylsilane sont ajoutés goutte à goutte pendant 1 h. Le milieu réactionnel est agité à température ambiante pendant 20 min puis filtré sur célite (rincée avec du méthanol chaud). Le filtrat est concentré pour conduire au composé **21** sous la forme d'une huile incolore avec un rendement de 95% (390 mg ; 0,75 mmol).

Formule chimique **21** : C₁₈H₃₁BrO₁₂

Masse molaire : 519,34g.mol⁻¹
R_{f} : 0,40 [DCM/MeOH (85:15)]

### Préparation du 6,7-didésoxy-7-éthoxycarbonyl-α-D-manno-heptopyranosyl-(1→2)-α-D-mannopyranoside d'azidoéthyle 22 :

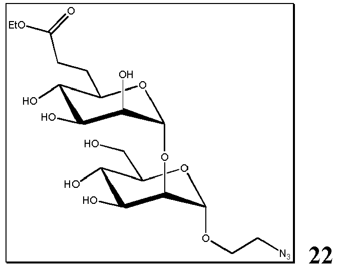

440 mg (1 éq ; 0,85 mmol) du composé **21** sont dissous dans 3,5 mL de DMF fraîchement distillé. 66 mg (1,2 éq ; 1,02 mmol) d'azoture de sodium sont ajoutés. La solution est agitée à température ambiante pendant 5 jours puis évaporée. Le résidu obtenu est purifié par chromatographie flash sur gel de silice (AcOEt/MeOH : 10% (210 mL) ; 20%) pour conduire au composé **22** sous la forme d'un solide blanc avec un rendement de 99% (407 mg ; 0,85 mmol).

Formule chimique **22** : C₁₈H₃₁N₃O₁₂

Masse molaire : 481,45 g.mol⁻¹
R_{f} : 0,43 [AcOEt/MeOH (8:2)]
SM, ESI⁺ m/z : 482 [M+H]⁺

### Préparation du 6,7-didésoxy-7-hydroxycarbonyl-α-D-manno-heptopyranosyl-(1→2)-α-D-mannopyranoside d'azidoéthyle 23:

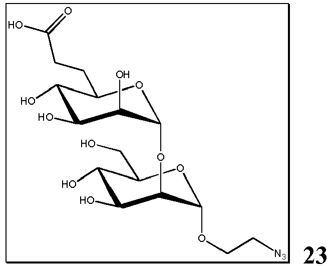

377 mg (1 éq ; 0,78 mmol) du composé **22** sont dissous dans 932 µL (1,2 éq ; 0,932 mmol) d'une solution de soude 1 N. La solution est agitée à température ambiante pendant 20 h puis concentrée. Le résidu obtenu est purifié par chromatographie flash sur gel de silice (isopropanol/ammoniaque 35%/eau : 6/3/1) pour conduire au composé **23** sous la forme d'un solide blanc avec un rendement de 86% (316 mg ; 0,67 mmol).

Formule chimique **23** : C₁₆H₂₇N₃O₁₂

Masse molaire : 453,40 g.mol⁻¹
R_{f} : 0,38 [isopropanol/NH₄OH/eau (6:3:1)]
SM, ESI⁺ m/z : 454 [M+H]⁺

### Préparation du 6,7-didésoxy-7-hydroxycarbonyl-α-D-manno-heptopyranosyl-(1→2)-α-D-mannopyranoside d'aminoéthyle (M6C-α(1,2)-Man-Ethyl-NH₂) 24:

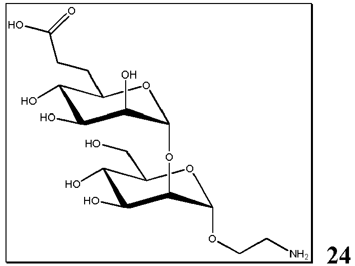

316 mg (1 éq ; 0,67 mmol) du composé **23** sont dissous dans 15 mL d'un mélange méthanol/eau 2:1. 32 mg (10% massique) de palladium sur charbon à 10% sont ajoutés. 536 µL (5 éq ; 3,36 mmol) de triéthylsilane sont ajoutés goutte à goutte pendant 40 min. Après 45 min d'agitation à température ambiante, il reste du produit de départ. 536 µL (5 éq ; 3,36 mmol) de triéthylsilane sont ajoutés goutte à goutte pendant 30 min. Le milieu réactionnel est agité à température ambiante pendant 10 min (pas d'évolution) puis filtré sur célite (rincée avec du méthanol chaud). Le filtrat est concentré puis dissous dans 3 mL d'un mélange méthanol/eau 2:1. 60 mg (20% massique) de palladium sur charbon à 10% sont ajoutés. 1,07 mL (10 éq ; 6,72 mmol) de triéthylsilane sont ajoutés goutte à goutte pendant 20 min. La suspension est agitée à température ambiante pendant 30 min puis filtrée sur célite (rincée avec du méthanol chaud). Le filtrat est concentré pour conduire au composé **24** sous la forme d'une huile incolore avec un rendement de 97 % (280 mg ; 0,66 mmol).

Formule chimique **24** : C₁₆H₂₉NO₁₂

Masse molaire : 427,40 g.mol⁻¹
R_{f} : 0,51 [isopropanol/NH₄OH/eau (5:4:1)]
SM, ESI⁺ m/z : 428 [M+H]⁺

### Préparation du 6,7-didésoxy-7-hydroxycarbonyl-α-D-manno-heptopyranosyl-(1 →2)-α-D-mannopyranoside de (4-éthoxy-2,3-dioxocyclobut-1-ényl)aminoéthyle (M6C-α(1,2)-Man-EthylSq) 25:

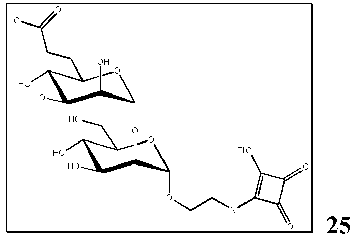

45 µL (1 éq; 0,307 mmol) de squarate de diéthyle sont dilués dans 300 µL d'un mélange éthanol/eau 2 :1. Le pH de la solution est de 5.20 µL (0,5 éq ; 0,154 mmol) de triéthylamine sont ajoutés pour arriver à pH 8-9. 131 mg (1 éq ; 0,307 mmol) du composé **24** dissous dans 1,7 mL d'un mélange éthanol/eau 2:1 sont ajoutés goutte à goutte. La solution est agitée à température ambiante pendant 2h30, en maintenant le pH à 8-9 par ajout de triéthylamine, puis concentrée. Le résidu obtenu est purifié par chromatographie flash sur gel de silice (AcOEt/MeOH : dépôt 3:7; élution : 30% (20 mL) ; 40% (10 mL) ; 50% (20 mL) ; 70% (30 mL) pour conduire au composé **25** sous la forme d'un solide incolore avec un rendement de 73% (123 mg ; 0,223 mmol).

Formule chimique **25** : C₂₂H₃₃NO₁₅

Masse molaire : 551,50 g.mol⁻¹
R_{f} : 0,36 [AcOEt/MeOH (1:1)]
SM, ESI⁺ m/z : 552 [M+H]⁺

### EXEMPLE 2 :

### Etude des analogues disaccharidiques 18, 19, 24 et 25 de l'invention, répondant à la formule générale (I)

### 1) Liaison des analogues M6P de l'invention avec le RM6P-CI

Les plaques de 96 puits (Maxisorp Nunc) sont incubées une nuit à 4°C avec 200 µL de PMP (penta mannose 6-phosphate) à la concentration de 200 ug.mL¹, dans le tampon carbonate (NaHCO₃/Na₂CO₃ à 0,1M, pH 9,6). Le lendemain, la solution contenant le PMP résiduel est écartée et les puits sont saturés, pendant 1 h à température ambiante, avec 360 µL de la gélatine 1% (*Type A from Porcine Skin*) diluée dans le PBS (1,9 mM NaH₂PO₄, 8,1 mM Na₂PO₄ et 154 mM NaCl, pH 7,4). Les puits sont ensuite rincés 5 fois par le PBS additionné de 0,2% de gélatine. Tous les lavages ainsi que les dilutions sont réalisés dans la solution de PBS additionné de 0,2% de gélatine. Les analogues M6P à tester aux différentes concentrations (de 10⁻² à 10⁻⁷ M) sont pré-incubés en présence du RM6P-CI préalablement biotinylé (RM6P*b*) (2,5 µg.mL⁻¹ ) pendant 20 min, puis 200 µL du mélange sont incubés dans les puits pendant 2 h, à température ambiante. Après 3 lavages, les puits sont incubés pendant 1 h avec une solution de streptavidine-peroxydase (250 µL par puits ; 3.10⁻⁸ M). Après 3 autres lavages, 200 µL d'une solution d'OPD (o-phenylènediamine 1 mg.mL⁻¹ dans le tampon citrate pH 5,0 et 1 µL 30% H₂O₂.mL⁻¹ ; Sigma Aldrich) est ajoutée. Après 20 min d'incubation au noir et à température ambiante, les densités optiques sont mesurées à la longueur d'onde de 450 nm.

Les affinités des disaccharides phosphonate **18** et carboxylate **24** de formule (I) de l'invention ont été mesurées et comparées avec celles de leurs homologues respectifs monosaccharidiques.

L'affinité désigne la capacité de fixation (en l'occurrence par l'intermédiaire d'une liaison covalente) des analogues du M6P pour le RM6P-CI.

L'affinité relative permet de comparer l'affinité des molécules avec le **M6C-Ethyl-NH₂** pris comme référence et pour lequel une valeur égale à 1 lui est attribuée.

Les résultats obtenus sont résumés dans le tableau 1 ci-dessous.

L'affinité du disaccharide phosphonate **18** (ligne 1 du tab. 1), le 6-désoxy-6-dihydroxyphosphinylméthylène-α-D-mannopyranose-α-(1,2)-D-mannopyrannose d'aminoéthyl, représenté par M6Pn-α(1,2)-Man-Ethyl-NH₂, est donc comparée à celle du monosaccharide phosphonate (ligne 2), représenté M6Pn-Ethyl-NH₂.

L'affinité du disaccharide carboxylate **24** (ligne 3), le 6,7-didésoxy-7-hydroxycarbonyl-α-D-*manno*-hept-6-anopyranose-α-(1,2)-α-D-mannopyrannose d'aminoéthyle, représenté par M6C-α(1,2)-Man-Ethyl-NH₂, est donc comparée à celle du monosaccharide carboxylate (ligne 4), représenté M6C-Ethyl-NH₂.

**Tableau 1**

| Analogues M6P | Structure chimique | IC₅₀ en M | Affinité relative |
|---|---|---|---|
| **Composé 18 de l'invention** | 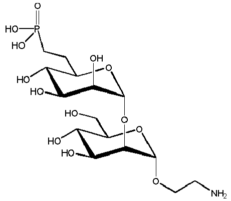 | | |
| M6Pn-α(1,2)-Man-Ethyl-NH₂ | | 0,5 x 10⁻⁵ M | 19,8 |
| M6Pn-Ethyl-NH₂ | 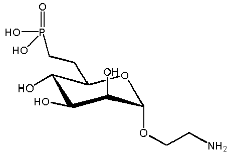 | 2,9 x 10⁻⁵ M | 3,4 |
| **Composé 24 de l'invention** | 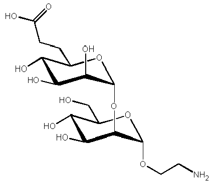 | | |
| M6C-α(1,2)-Man-Ethyl-NH₂ | | 1,7 x 10⁻⁵ M | 5,8 |
| M6C-Ethyl-NH₂ | 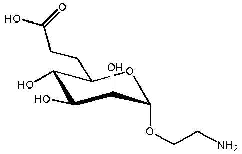 | 9,9 x 10⁻⁵ M | 1 |

### Conclusion :

Une meilleure affinité des analogues disaccharidiques **18** et **24** de l'invention est observée pour le RM6P-CI que celle des mêmes analogues monosaccharidiques. De plus, un gain d'affinité de l'analogue phosphonate **18** est observé par rapport à l'analogue carboxylate **24,** aussi bien en série disaccharidique qu'en série monosaccharidique.

### 2) Evaluation de la cytotoxicité des analogues M6P de l'invention avec RM6P-CI

La cytotoxicité des analogues disaccharidiques phosphonates **18, 19** et carboxylates **24, 25** de l'invention a été évaluée sur des cellules de cancers de la prostate LNCaP, et a été comparée à celle de leurs homologues monosaccharidiques respectifs.

Les analogues disaccharidiques **18, 19, 24** et **25** de formule (I) de l'invention ainsi que les analogues monosaccharidiques des analogues disaccharidiques 19, 24 et 25 sont testés :
M6Pn-α(1,2)-Man-Ethyl-NH₂ (compo **18**) ;
M6Pn-α(1,2)-Man-EthylSq (compo **19)** ;
M6Pn-EthylSq (décrit ci-dessous) ;
M6C-α(1,2)-Man-Ethyl-NH₂ (compo **24**) ;
M6C-Ethyl-NH₂ (décrit dans le tableau 1) ;
M6C-α(1,2)-Man-EthylSq (compo **25**) ;
M6C-EthylSq (décrit ci-dessous).

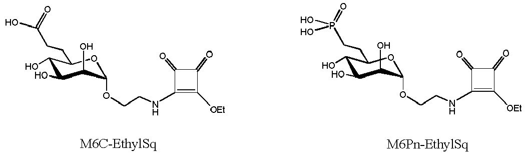

### Protocole expérimental

Les cellules humaines de cancer de la prostate LNCaP sont incubées avec des solutions des analogues tels que décrits ci-dessus présentant des concentrations allant de 10⁻³ M à 10⁻⁷ M.

La survie cellulaire est mesurée après 4 jours d'incubation à l'aide d'un test MTS (3-(4,5-diméthylthiazol-2-yl)-5-(3-carboxyméthoxyphényl)-2-(4-sulfophényl)-2H-tétrazolium). Ce dernier est réduit par une enzyme mitochondriale et va former un composé de couleur jaune orangée qui est soluble en milieu aqueux. La mesure des densités optiques indique ainsi la quantité de cellules vivantes. Les résultats de la croissance cellulaire ont été déterminés par rapport à un contrôle traité avec uniquement le véhicule (solution dans laquelle sont dilués les analogues).

Les résultats obtenus sont représentés à la **figure 1****.**

### Conclusion :

Aucun des composés testés ne présente de cytotoxicité significative sur les cellules humaines LNCaP même à des concentrations très élevées (jusqu'à 10⁻³ M).

Ainsi les analogues M6P testés ne présentent pas de toxicité intrinsèque pour des cellules exprimant le RM6P-CI telles que les cellules LNCaP.

### 3) Fonctionnalisation de nanoparticules pour la thérapie photodynamique monophotonique

Un composé d'intérêt Ⓨ, à savoir des nanoparticules de silice mésoporeuse incorporant un photosensibilisateur de type porphyrine neutre, est ensuite fonctionnalisé en surface avec les analogues :
- disaccharidiques phosphonates M6Pn-α(1,2)-Man-EthylSq (**19**) et carboxylates M6C-α(1,2)-Man-EthylSq (**25**) ;
- monosaccharidiques phosphonate M6Pn-EthylSq et carboxylate M6C-EthylSq.

La structure de la porphyrine neutre utilisée pour la thérapie photodynamique est représentée ci-dessous :

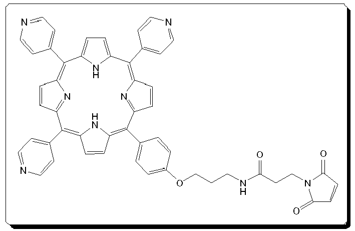

### Préparation des nanoparticules de silice incorporant la porphyrine

La porphyrine doit porter un groupement trialkoxysilane qui permettra son incorporation dans le réseau silicique lors du procédé sol-gel décrit ci-après. Pour cela, la porphyrine est mise en réaction avec le 3-mercaptopropyltriméthoxysilane dans le méthanol à température ambiante pendant 12h.

Le CTAB (bromure de cétyltriméthylammonium) est dissous dans une solution de soude à 0,2 M. Des micelles vont ainsi se former dans la solution. La porphyrine silylée puis le TEOS (orthosilicate de tétraéthyle) sont ajoutés. Rapidement, le milieu réactionnel est dilué avec un grand volume d'eau afin d'abaisser le pH à 8-8,5 et d'initier la condensation. Après 6 min, le milieu réactionnel est neutralisé à l'aide d'une solution d'HCl à 0,2 M. Les nanoparticules sont obtenues après des étapes de lavages avec du nitrate d'ammonium pour enlever le CTAB contenu dans les pores.

Lors de la synthèse des nanoparticules, les temps de réaction ainsi que le pH doivent être contrôlés minutieusement afin de maîtriser la taille des nanoparticules formées. Les nanoparticules ainsi obtenues présentent un diamètre hydrodynamique d'environ 200 nm et contiennent 11 µg de porphyrine par gramme de nanoparticule.

### Fonctionnalisation de la surface des nanoparticules

Ces nanoparticules sont ensuite fonctionnalisées en surface avec les analogues monosaccharidiques carboxylate M6CEthylSq et phosphonate M6PnEthylSq et les analogues disaccharidiques carboxylate M6C-α(1,2)-Man-EthylSq (**25**) et phosphonate M6Pn-α(1,2)-Man-EthylSq (**19**).

La fonctionnalisation se déroule en deux temps : la première étape consiste à introduire des fonctions amines sur la surface des nanoparticules qui, dans la seconde étape, viendront substituer le groupement éthoxy présent sur le squarate du bras de l'analogue. Les nanoparticules sont mises en réaction avec de l'aminopropyltriéthoxysilane (APTS) dans un mélange eau/éthanol (2:1). Le pH du milieu réactionnel est ajusté à 6 par ajout d'une solution d'HCl à 0,2 M puis agitée à température ambiante pendant 20 h. Les nanoparticules sont obtenues après plusieurs étapes de lavages à l'éthanol suivis de centrifugations.

Pour le couplage des analogues carboxylates (M6CEthylSq et M6C-α(1,2)-Man-EthylSq (**25**)), l'analogue a été dissout dans un mélange éthanol/eau (1:1) en présence des nanoparticules puis la suspension a été chauffée à 50°C pendant 12 h.

Il est à noter que de la triéthylamine peut être ajoutée pour maintenir le pH à 8-9 et favoriser le couplage des analogues aux nanoparticules.

Après des étapes de lavages à l'eau et à l'éthanol, les nanoparticules fonctionnalisées avec les analogues carboxylates sont obtenues.

Le couplage (greffage) de l'analogue disaccharidique carboxylate M6C-α(1,2)-Man-EthylSq (**25**) avec les nanoparticules de silice incorporant la porphyrine (représentée par une étoile) est représenté ci-dessous :

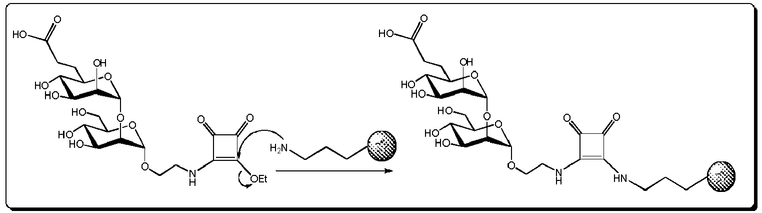

On obtient ainsi un conjugué de formule (III).

Le groupement squarate étant observable en UV, le dosage de la quantité de monosaccharides et disaccharides a été réalisé par spectroscopie UV/visible.

Le tableau 2 ci-dessous représente la quantité de porphyrine incorporée (en µg/g) et la quantité de monosaccharide ou disaccharide (en (µmol/g) greffé sur les nanoparticules.

L'abréviation « MSN » représente les nanoparticules de silice mésoporeuse seules, sans greffage avec le monosaccharide ou le disaccharide.

Les analogues M6CEthylSq, M6C-α(1,2)-Man-EthylSq (25) et M6Pn-α(1,2)-Man-EthylSq (**19**) greffés avec les nanoparticules de silice mésoporeuse sont représentés par MSN-M6C-EthylSq; MSN-M6C-α(1,2)-Man-EthylSq et MSN-M6Pn-α(1,2)-Man-EthylSq. Ces composés sont des conjugués répondant à la formule générale (III).

**Tableau 2**

| Nanoparticules | Quantité de porphyrine (µmol/g) | Quantité de mono- ou di-saccharide (µmol/g) |
|---|---|---|
| MSN | 11 µmol/g | - |
| MSN-M6C-EthylSq | 11 µmol/g | 351 µmol/g |
| MSN-M6C-α(1,2)-Man-EthylSq | 11 µmol/g | 329 µmol/g |
| MSN-M6Pn-α(1,2)-Man-EthylSq. | 11 µmol/g | 167 µmol/g |

### Evaluation in vitro en thérapie photodynamique

Les conjugués carboxylates MSN-M6C-EthylSq et MSN-M6C-α(1,2)-Man-EthylSq de formule (III) ont été utilisés dans des expériences de PDT *in vitro* sur des cellules de cancer de la prostate LNCaP qui surexpriment le RM6P-CI.

Les cellules LNCaP ont été incubées avec 80 µg.mL⁻¹ de nanoparticules seules (MSN) ou avec 80 µg.mL⁻¹ de nanoparticules fonctionnalisées avec le mono- ou disaccharide (i.e. avec 80 µg.mL⁻¹ de conjugués **MSN-M6C-EthylSq** et **MSN-M6C-**α**(1,2)-Man-EthylSq)** pendant 3, 6, 9 ou 18 h puis irradiées pendant 20 min avec un laser à 650 nm (3 mW, 11,25 J.cm-²).

Le test de survie cellulaire MTS a été effectué 48 h après l'irradiation.

Après 3 heures d'incubation, une très légère augmentation de l'effet phototoxique des nanoparticules fonctionnalisées avec les mono- ou di-saccharides est observée.

Lorsque le temps d'incubation est augmenté à 6 h on note que la fonctionnalisation par un disaccharide carboxylate apporte une forte amélioration de l'effet phototoxique, avec 73% de mort cellulaire contre 35% pour le monosaccharide carboxylate. Ceci est lié à l'amélioration de la reconnaissance des RM6P-CI conduisant à une internalisation plus rapide des nanoparticules (fonctionnalisées avec un disaccharide) dans les cellules.

Ces résultats mettent en évidence l'avantage de la fonctionnalisation des nanoparticules avec des analogues disaccharidiques, permettant à la fois de réduire le temps d'incubation et d'augmenter considérablement l'effet phototoxique des nano-outils.

En augmentant le temps d'incubation à 9 h la mort cellulaire obtenue pour les cellules traitées avec les nanoparticules fonctionnalisées avec le disaccharide atteint 81% contre 55% avec les nanoparticules fonctionnalisées avec le monosaccharide.

Dans le cas d'une incubation pendant 18h une amélioration de l'effet phototoxique est observée pour les nanoparticules fonctionnalisées avec les analogues carboxylates monosaccharidiques par rapport à l'effet de ces mêmes nanoparticules après une incubation de 9h.

En revanche l'effet des nanoparticules disaccharidiques n'est pas augmenté à 18h car cet effet est déjà maximal après 9h d'incubation.

Les effets phototoxiques des nanoparticules seules (MSN) et fonctionnalisées avec le monosaccharide carboxylate (MSN-M6C-EthylSq) et disaccharide carboxylate (MSN-M6C-α(1,2)-Man-EthylSq) aux différents temps d'incubation sont récapitulés dans la **figure 2****.**

### Conclusion

La fonctionnalisation de nanoparticules avec des dimannosides analogues du M6P permet donc d'augmenter très significativement l'efficacité phototoxique par rapport aux nanoparticules greffées avec les analogues monosaccharidiques tout en réduisant le temps d'incubation. Cette augmentation d'efficacité associée à l'affinité pour le RM6P-CI peut être déterminante pour l'application diagnostique ou thérapeutique des composés d'intérêt Ⓨ (nanoparticules, glycoprotéines, petites molécules, ...) dont la clairance métabolique chez l'homme peut être rapide.

### EXEMPLE 3 :

### Synthèse de composés de formule (I)

### Préparation de disaccharides 26, 27 et 28 répondant à la formule (I) dans laquelle X représente le groupement phosphonate

Le procédé de préparation de disaccharides **26, 27** et **28** répondant à la formule (**I**) dans laquelle X représente le groupement phosphonate est illustré ci-dessous :

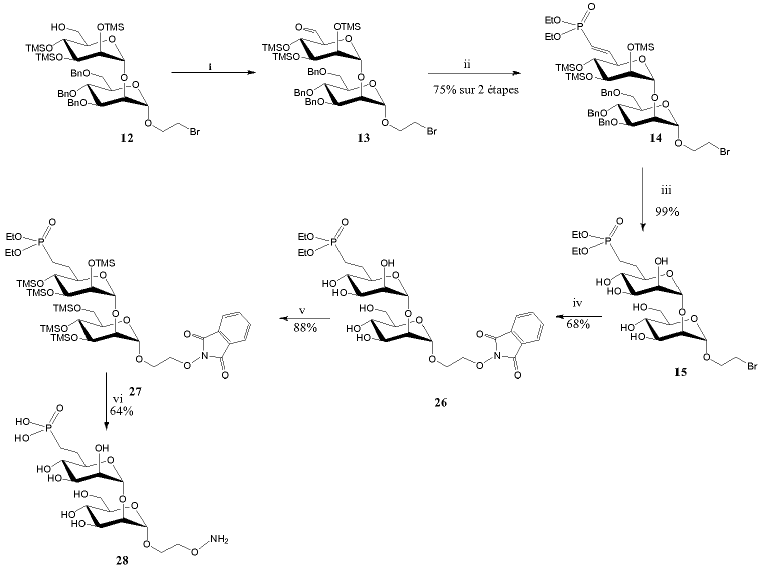

Le début de la synthèse du disaccharide phosphonate **28** de formule (I) est identique à celle du disaccharide phosphonate **19,** à savoir le phosphonate **15** est obtenu à partir de l'alcool **12** de la même manière que décrit ci-dessus.

Pour synthétiser le disaccharide phosphonate **28** de formule (I), l'aldéhyde **13** est mis en réaction avec le méthylènediphosphonate de tétraéthyle, préalablement déprotoné par de l'hydrure de sodium, pour former le composé **14** avec un rendement de 75% sur deux étapes. Lors d'une étape d'hydrogénation catalytique, en présence de palladium sur charbon et pour laquelle l'hydrogène est généré in situ par ajout progressif de triéthylsilane, la double liaison ainsi que les trois benzyles sont réduits et les groupements triméthylsilyles sont hydrolysés. On obtient ainsi le composé **15,** sans purification, avec un rendement quantitatif. L'atome de brome est ensuite substitué par du *N*-hydroxyphthalimide pour former l'intermédiaire **26** avec un rendement de 68%. Les fonctions alcools sont ensuite protégées par du chlorure de triméthylsilyle pour conduire au composé **27** avec un rendement de 88%. L'intermédiaire **27** ainsi obtenu, sans purification est ensuite mis en réaction avec du chlorure de triméthylsilyle et de l'iodure de sodium pour former le phosphonate bis(triméthylsilylé) par une réaction de type Rabinowitz. Cet intermédiaire est ensuite converti en acide phosphonique par action de l'hydrazine monohydratée dans le méthanol qui va aussi déplacer les groupements triméthylsilyles présents sur les alcools secondaires du sucre et réagir avec l'hydroxyphtalimide afin de démasquer la fonction oxyamine. On obtient ainsi, en une seule étape, le composé **28** déprotégé sur la partie phosphonate, sur les alcools des deux sucres et portant en position anomère la fonction oxyamine, nécessaire pour le couplage avec l'enzyme, avec un rendement de 64% sur deux étapes.

Conditions et réactifs : (i) Dess-Martin periodinane, DCM, TA, 4h (ii) méthylènediphosphonate de tétraéthyle, NaH, THF, TA, 45 min; (iii) Pd/C, Et₃SiH, MeOH ; (iv) *N*-hydroxyphtalimide, NaH, DMF, 65°C, 20 h ; (v) TMSC1, NEt₃, DCM, DMF, TA, 24h; (vi)-a TMSCl, NaI, Et₃N, ACN, 35°C, 4 h ; (vi)-b N₂H₄.H₂O, MeOH, TA, une nuit.

### Partie expérimentale

### Préparation du 6-désoxy-6-diéthoxyphosphinylméthylène-α-D-mannopyranosyl-(1→2)-α-D-mannopyranoside de 2-N-oxyphtalimidoéthyle 26:

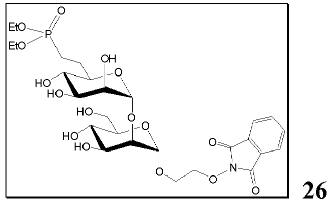

177 mg (2,2 éq, 5,3 mmol) du NaH sont suspendus dans 80 mL de DMF. 783 mg (2 éq, 4,8 mmol) de *N*-hydroxyphthalimide sont ajoutés (solution rouge). Après une heure d'agitation à 65°C (solution rouge foncée), 1,4 g (1 éq, 2,4 mmol) du composé **15** dissous dans 20 mL du DMF sont ajoutés. Après 20 h à 65°C, la solution est refroidie et le DMF est évaporé sous pression réduite. Le brut obtenu est purifié sur une colonne de gel de silice avec un gradient AcOEt/MeOH (95:5 →80:20) pour donner le composé **26** avec un rendement de 68% (1,08, 1,62 mmol).

Formule chimique **26** : C₂₇H₄₀NO₁₆P

Masse molaire : 665,58 g.mol⁻¹
R_{f} : 0,6 [AcOEt/MeOH (70:30)]

### Préparation du 2,3,4-tri-O-triméthylsilyl-6-désoxy-6-diéthoxyphosphinylméthylène-α-D-mannopyranosyl-(1→2)-3,4,6-tri-O-triméthylsilyl-α-D-mannopyranoside de 2-N-oxyphtalimidoéthyle 27 :

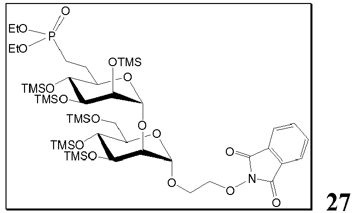

1,05 g (1 éq, 1,6 mmol) du composé **26** sont dissous dans 10 mL de DCM fraîchement distillé et 6,6 mL (47,3 mmol, 30 éq) de triéthylamine. Le produit de départ n'est pas complètement soluble. 2,4 mL (18,9 mmol, 12 éq) de chlorure de triméthylsilyle sont ajoutés goutte à goutte. La solution est agitée à température ambiante pendant 25 h puis évaporée. Au cours de la réaction, la solution devient marron. Le résidu marron est dissous dans du cyclohexane puis filtré sur célite. Le filtrat est concentré pour conduire au composé **27** avec un rendement de 88% (1,52 g; 1,38 mmol) directement utilisé pour l'étape suivante.

Formule chimique **27 :** C₄₅H₈₈NO₁₆Si₆

Masse molaire : 1098,67 g.mol⁻¹
R_{f} : 0,57 [DCM/Et₂O (90:10)]

### Préparation du 6-désoxy-6-dihydroxyphosphinylméthylène-α-D-mannopyranosyl-(1→2)-D-mannopyranoside d'aminooxyéthyle 28 :

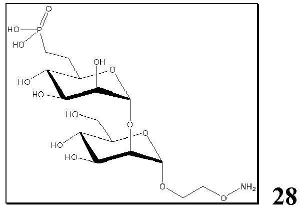

A une solution du composé **27** (1 éq, 367 mg, 0,33 mmol) dans 11 mL de CH₃CN anhydre sont ajoutés Et₃N (7,2 éq, 0,34 ml, 2,4 mmol), TMSCl (7 éq, 0,296 mL, 2,34 mmol) et NaI (7 éq, 350 mg, 2,34 mmol) sous atmosphère d'azote. Après 4 h à température ambiante, l'agitation est arrêtée. Le surnageant est canulé et le sel résiduel est rincé avec CH₃CN anhydre (2x3 mL) puis le solvant est canulé. Le solvant est évaporé et le résidu obtenu est repris dans 7,5 mL de méthanol anhydre contenant de l'hydrazine (12 éq, 4,01 mmol, 0,194 mL). La réaction est laissée pendant une nuit sous agitation. Un précipité blanc est formé. Le résidu obtenu après évaporation est purifié par chromatographie sur colonne de gel de silice (éluant : isopropanol/NH₄OH/H₂O 5:4:1) pour conduire au composé **28** avec un rendement de 64% (103 mg, 0,22 mmol).

Formule chimique **28** : C₁₅H₃₀NO₁₄P

Masse exacte : 479,37 g.mol⁻¹
R_{f} : 0,33 [isopropanol/NH₄OH/H₂O (5:4:1)]
SM, ESI⁺ m/z : 480,3 [M+H]⁺

### EXEMPLE 4 :

### Etude de l'analogue disaccharidique 28 de l'invention, répondant à la formule générale (I)

### 1) Fonctionnalisation d'une enzyme lysosomale, alpha glucosidase acide (GAA) par l'analogue disaccharidique (28)

Un composé d'intérêt Ⓨ, à savoir une enzyme lysosomale, alpha glucosidase acide (GAA) qui peut être utilisée pour le traitement de la maladie de Pompe ou glycogénose de type II⁷⁻⁸ est fonctionnalisée sur les chaînes oligosaccharidiques avec l'analogue disaccharidique phosphonate M6Pn-α(1,2)-Man-Ethyloxyamino (**28**). La maladie de Pompe est représentative de la majorité des 53 différentes maladies lysosomales dont l'efficacité de traitement par enzymothérapie de substitution dépend du RM6P-CI⁷⁻⁹ . En effet, ce récepteur joue un rôle clé pour l'internalisation cellulaire des enzymes recombinantes injectées par voie intraveineuse et pour leur routage intracellulaire jusqu'aux lysosomes.

### Protocole expérimental :

La GAA humaine recombinante (rhGAA) est produite dans le système de cellules eucaryotes CHO capables de produire des M6P à l'extrémité des chaînes glycosylées et est purifiée à partir du milieu de culture. Le poids moléculaire de l'enzyme est d'environ 110 000 Da et elle est reconnue par un anticorps spécifique anti-GAA humaine (anti-LYAG, Genetex). La GAA humaine recombinante peut être aussi produite dans le système baculovirus/cellules d'insecte Sf9 qui ne produit pas de résidus M6P et qui produit des chaînes oligosaccharidiques mannosylées.

La rhGAA est ensuite fonctionnalisée sur les chaînes oligosaccharidiques avec les analogues disaccharidiques phosphonate M6Pn-α(1,2)-Man-Ethyloxyamino (**28**). La fonctionnalisation se déroule en deux temps : la première étape consiste à générer des fonctions aldéhydes par l'oxydation des mannoses qui, dans la seconde étape, viendront réagir avec les fonctions oxyamines présentes sur le bras éthyloxyamino de l'analogue (**28**). Les enzymes sont oxydées par 1 mM NaIO₄ pendant 30 min à 4°C puis mises en réaction avec les analogues disaccharidiques (100 équivalents pour 1 enzyme) pendant 2 h à 25°C. Les enzymes fonctionnalisées sont obtenues après dialyse afin d'éliminer les analogues n'ayant pas réagi.

Le couplage (greffage) de l'analogue disaccharidique phosphonate M6Pn-α(1,2)-Man-Ethyloxyamino (**28**) avec les enzymes est représenté ci-dessous :

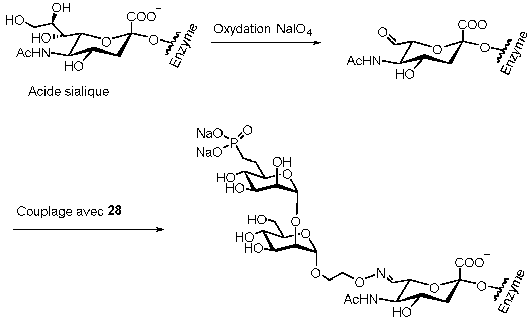

L'oxydation de l'acide sialique est effectuée par le périodate de sodium.

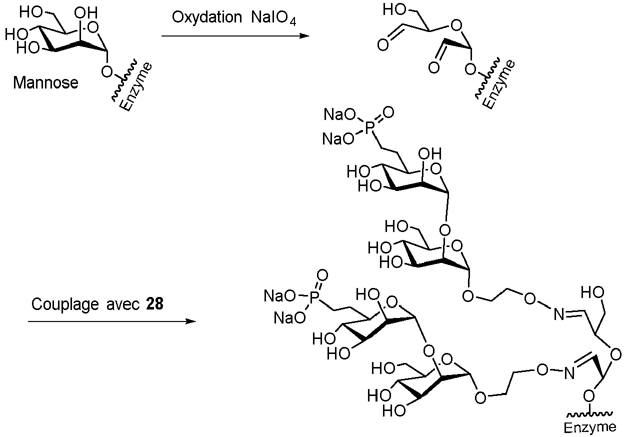

L'oxydation du mannose est effectuée par le périodate de sodium.

### 2) Affinité pour le RM6P-CI de la rhGAA fonctionnalisée par M6Pn-α(1,2)-Man-Ethyloxyamino (28)

Le conjugué de formule (III), à savoir la rhGAA-fonctionnalisée par l'analogue disaccharidique phosphonate M6Pn-α(1,2)-Man-Ethyloxyamino (**28**) est désigné ci-après par rhGAA-M6Pn-α(1,2)-Man-Ethyloxyamino (**28**).

Le conjugué de formule (III) est évalué pour son affinité avec RM6P-CI. L'affinité de liaison de rhGAA et du conjugué rhGAA-M6Pn-α(1,2)-Man-Ethyloxyamino (**28**) a été évaluée par une technique de compétition de liaison compétitive avec le RM6P-CI décrite dans l'exemple 2 et comparée à l'affinité du M6P.

Les données indiquent une forte affinité pour rhGAA-M6Pn-α(1,2)-Man-Ethyloxyamino (**28**) (conjugué de formule (III)) correspondant à une concentration inhibitrice à 50% (IC₅₀) de 0,55 10⁻⁷ M alors que l'affinité de la rhGAA était de 3 10⁻⁷ M soit 5,5 fois inférieure. Par comparaison l'affinité du M6P est de 3 M 10⁻⁵ dans ces mêmes expériences. Ce résultat indique le couplage efficace de plusieurs dimannosides analogues du M6P sur les chaînes glycosylées de l'enzyme.

### 3) Internalisation du conjugué rhGAA-M6Pn-α(1,2)-Man-Ethyloxyamino (28) (conjugué de formule (III)) dans les myoblastes de patients atteints de la forme adulte de la maladie de Pompe (figure 3)

### Protocole expérimental :

Les cellules de type myoblastes provenant d'un malade adulte atteint par la maladie de Pompe sont maintenues en culture primaire.

L'activité catalytique de la GAA est mesurée dans les extraits cellulaires par le substrat synthétique 4-méthylumbelliferyl-α-D-glucopyranoside (4-MUG). Ce substrat et les standards de poids moléculaire sont obtenus chez Sigma. L'activité de la GAA dans les extraits cellulaires (20 µl) est mesurée dans un volume réactionnel de 75 µl contenant 50 mM acide citrique, 115 mM K₂HPO₄, 110 mM KCl, 10 mM NaCl, pH 5.0, avec 6 mM 4-MUG pendant 10 min à température ambiante. La réaction est arrêtée avec 75 µL 0.1 M Tris HCl pH 8. La fluorescence est lue avec des filtres d'excitation à 355 nm et d'émission à 460 nm dans des plaques de 96-puits et comparée à une courbe standard obtenue avec la 4-méthylumbelliferone. L'activité catalytique est exprimée par mg de protéine. Moyenne ± écart-type.

Il ressort de la **figure 3** que le greffage des disaccharides augmente significativement la pénétration cellulaire de la GAA dont la concentration est mesurée par son activité catalytique sur le substrat 4-MUG.

### Conclusion :

Le produit d'intérêt rhGAA fonctionnalisé par l'addition de disaccharides (**28**) est plus efficace dans le traitement d'un déficit enzymatique lié à la maladie de Pompe.

### 4) Etude du processus de maturation cellulaire dans les myoblastes d'un patient adulte atteint de la maladie de Pompe du conjugué rhGAA-M6Pn-α(1,2)-Man-Ethyloxyamino (28) (conjugué de formule (III)) (figure 4)

La maturation cellulaire de la GAA¹⁰ est réalisée dans les endosomes et lysosomes par plusieurs coupures enzymatiques spécifiques qui transforment successivement le précurseur inactif de 110 kDa en formes intermédiaires de 95 kDa et 76 kDa puis en une forme mature et active de 60-70 kDa dans les lysosomes¹⁰.

Les myoblastes sont incubés 48 h en présence de 50 nM rhGAA ou du conjugué rhGAA-M6Pn-α(1,2)-Man-Ethyloxyamino (**28**) (conjugué de formule (III)) puis lysés et les protéines extraites. La maturation cellulaire de l'enzyme GAA internalisée dans les myoblastes de malades atteints de la maladie de Pompe est étudiée par la technique de séparation sur gel de polyacrylamide SDS suivie d'immunodétection de la GAA par Western blot avec un anticorps anti-GAA humaine (anti-LYAG, Genetex) ou anti-actine humaine (Invitrogen). L'actine est révélée en tant que protéine témoin indiquant que des quantités équivalentes de protéines sont déposées sur le gel. Cette expérience est représentative parmi 2 expériences. La flèche épaisse de la **figure 4** indique la forme intermédiaire de la GAA. La flèche noire indique la forme mature de la GAA (60-70 kDa).

La quantification de la forme mature de 60 kDa est réalisée en considérant comme 100% la bande présente chez les myoblastes provenant de sujet sain. L'expression de GAA 60 kDa dans les myoblastes de patient correspond à 1% de la GAA de sujet sain et n'est pas augmentée dans les myoblastes traités par rhGAA. A l'inverse, le traitement par le conjugué rhGAA-M6Pn-α(1,2)-Man-Ethyloxyamino (**28**) augmente la bande 60-70 kDa à 47% de la GAA d'un sujet sain. Ces résultats indiquent que le greffage des analogues disaccharides favorise très fortement le processus de maturation intracellulaire de la rhGAA conduisant à sa forme active de 60-70 kDa. Cette activité est importante pour une application dans le traitement par enzymothérapie de la maladie de Pompe mais peut aussi être applicable à l'ensemble des maladies lysosomales.

### Conclusion

La fonctionnalisation d'une enzyme lysosomale avec des dimannosides modifiés permet donc d'augmenter très significativement l'entrée cellulaire de cette enzyme par rapport à l'enzyme non greffée dans des cellules de patient atteint de la maladie de Pompe. La fonctionnalisation avec les dimannosides analogues du M6P permet de faciliter la maturation de l'enzyme à l'intérieur des endosomes et lysosomes par rapport à l'enzyme non fonctionnalisée. Cette augmentation d'efficacité associée à l'affinité pour le RM6P-CI peut être déterminante pour l'application diagnostique ou thérapeutique de glycoprotéines notamment pour la thérapie enzymatique de substitution utilisée pour les maladies de surcharge lysosomale ⁷⁻⁹.

### REFERENCES BIBLIOGRAPHIQUES

- 1.: Demande de brevet FR No. 14 50588 déposée le 23/01/14 ;
- 2.: Vidal S et al., Bioorg Med Chem. 10, 4051, (2002);
- 3.: Jeanjean A et al., Bioorg Med Chem. 14, 2575, (2006) ;
- 4.: Jeanjean A et al., Bioorg Med Chem Lett. 18, 6240, (2008) ;
- 5.: Demande internationale PCT/EP2010/059507 ;
- 6.: Distler JJ et al., J. Biol Chem, 32, 21687, (1991) ;
- 7.: Desnick, RJ, Schuchman EH, Nat Rev Genet 3, 954, (2002);
- 8.: Van der Ploeg AT, Reuser AJ, Lancet, 372, 1342, (2008);
- 9.: Hollak, CE, Wijburg FA, J Inherit Metab Dis, 37, 587, (2014);
- 10.: Moreland RJ et al., Gene, 491, 25, (2012).

## Revendications

1. Composé **caractérisé en ce qu'**il présente la formule générale (I) : 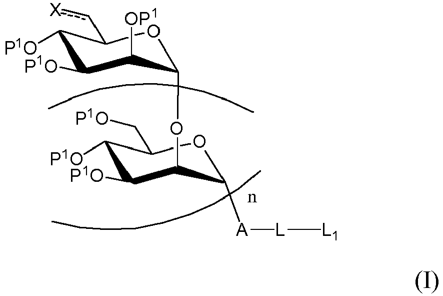 dans laquelle :
∘ n est un entier allant de 1 à 3,
∘ ------- représente une liaison simple ou une liaison double,
∘ chacun des P¹ représente indépendamment l'un de l'autre H, un groupe protecteur notamment choisi parmi (CH₃)₃Si- ; tBuMe₂Si- ; C₆H₅CH₂- ; 4-CH₃OC₆H₅CH₂- ; o-NO₂C₆H₅CH₂- ; CH₃CO- ; C₆H₅CO- ou CF₃CO- ;
∘ X représente :
∘∘ lorsque ------- est une liaison simple :
∘∘∘ le groupement phosphonate : 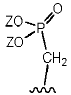
∘∘∘ le groupement fluoro phosphonate : 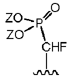
∘∘∘ le groupement bi-fluoro phosphonate : 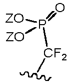
∘∘∘ le groupement carboxylate : 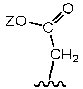
∘∘∘ le groupement malonate : 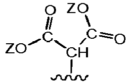
∘∘ lorsque ------- est une liaison double :
∘∘∘ le groupement phosphonate : 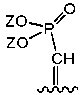
∘∘∘ le groupement carboxylate : 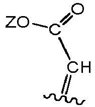 avec Z représentant indépendamment l'un de l'autre H ; un alkyle en C₁-C₅ ; CF₃CH₂- ; C₆H₅CH₂- ; C₆H₅- ; (CH₃)₃Si- ; un métal alcalin choisi parmi Na, Li ou K ; un ammonium NH₄,
∘ A représente un radical divalent choisi parmi -O-, -S-, -NH-, -CH₂-,
∘ L représente :
∘∘ -H ; -NH₂ ; -(CH₂)ₙ₁-CH=CH₂ ou -(CH₂)ₙ₁-C≡CH avec n₁ représentant un entier allant de 0 à 4, alors dans chacun de ces cas L₁ est absent,
∘∘ un radical hydrocarboné divalent saturé, linéaire ou ramifié, substitué ou non substitué, ayant de 1 à 30 atomes de carbone, un radical hydrocarboné divalent insaturé, linéaire ou ramifié, substitué ou non substitué, ayant de 2 à 30 atomes de carbone,
∘∘ un radical hydrocarboné divalent saturé ou insaturé tel que défini ci-dessus dont un ou plusieurs groupements -CH₂-, -CH=CH- et/ou -C=C- du radical hydrocarboné saturé ou insaturé est (sont) remplacé(s) indépendamment l'un de l'autre par un groupement -O- ; -NH-; -NR₁- avec R₁ représentant un alkyle en C₁-C₅ ; -S- ; -CO-NH- ; -NH-CO-O- ; -O-N=CH- ; -CO-NH-N=CH- ; un système cyclique ou hétérocyclique, saturé ou insaturé, substitué ou non ;
∘ L₁ représente :
-(CH₂)ₙ₁-CH=CH₂; -(CH₂)ₙ₁-C≡CH; -(CH₂)ₙ₁-N₃ ; -(CH₂)ₙ₁-SH ; -(CH₂)ₙ₁-NH₂ ;-(CH₂)ₙ₁-N=C=O ; -(CH₂)ₙ₁-N=C=S ; -(CH₂)ₙ₁-NHR₁ ; -(CH₂)ₙ₁-NR₁R₂ ; -(CH₂)ₙ₁-A₁-NH₂ ; -(CH₂)ₙ₁-A₁-NHR₁ ; -(CH₂)ₙ₁-A₁-NR₁R₂ ; -(CH₂)ₙ₁-NHCO-CH₂Hal ; -(CH₂)ₙ₁-COZ₁ ; -(CH₂)ₙ₁-A₁COZ₁ ; -(CH₂)ₙ₁-O-N=CH₂ ; -(CH₂)ₙ₁-CO-NH-N=CH₂ ; -(CH₂)ₙ₁-H ; un système cyclique ou hétérocyclique, saturé ou insaturé, substitué ou non ; un halogène choisi parmi F, Cl, Br ou I ; avec
n₁ tel que défini ci-dessus,
R₁ et R₂ représentant indépendamment l'un de l'autre un alkyle en C₁-C₅,
A₁ représentant -O-, -NH-,
Hal représentant Cl, Br ou I ;
Z₁ représentant -OH, -OR₁, -NHR₁, -NH-NH₂, -NH-NHR₁, -NH-NR₁R₂ avec R₁ et R₂ tels que définis ci-dessus, un halogène choisi parmi F, Cl, Br ou I.

2. Composé selon la revendication 1 **caractérisé en ce que** :
L représente :
∘ -NH₂, -(CH₂)ₙ₁-CH=CH₂ ou -(CH₂)ₙ₁-C=CH, avec n₁ tel que défini à la revendication 1, alors dans chacun de ces cas L₁ est absent,
∘ un radical hydrocarboné divalent saturé, linéaire ou ramifié, substitué ou non substitué, ayant de 1 à 10 atomes de carbone, un radical hydrocarboné divalent insaturé, linéaire ou ramifié, substitué ou non substitué, ayant de 2 à 10 atomes de carbone,
∘ un radical hydrocarboné divalent saturé ou insaturé tel que défini ci-dessus, dont un ou plusieurs groupements -CH₂-, -CH=CH- et/ou -C=C- du radical hydrocarboné saturé ou insaturé est(sont) remplacé(s) indépendamment l'un de l'autre par :
∘∘ un groupement -O- ; -NH- ; -S- ; -CO-NH- ; -NH-CO-O- ; et/ou
∘∘ un système cyclique ou hétérocyclique choisi parmi : 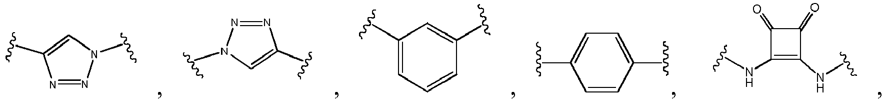 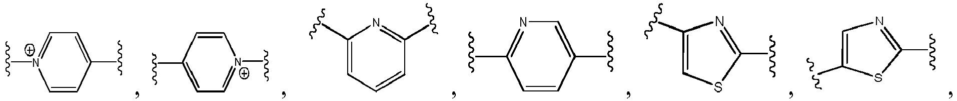 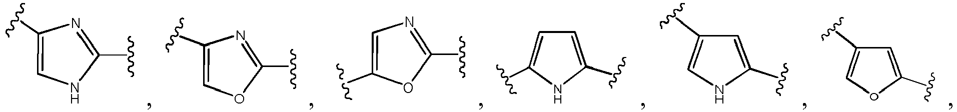 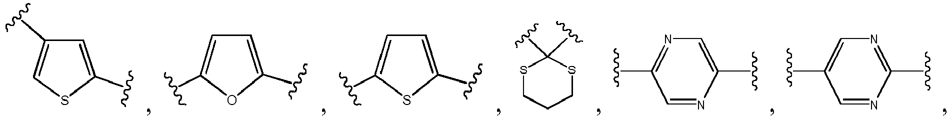 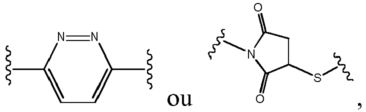
L₁ représente :
-(CH₂)ₙ₁-CH=CH₂ ; -(CH₂)ₙ₁-C≡CH ; -(CH₂)ₙ₁-N₃ ; -(CH₂)ₙ₁-SH ; -(CH₂)ₙ₁-NH₂ ;-(CH₂)ₙ₁-N=C=O; -(CH₂)ₙ₁-N=C=S ; -(CH₂)ₙ₁-O-NH₂ ; -(CH₂)ₙ₁-NHCO-CH₂Hal;-(CH₂)ₙ₁-COOH ; -(CH₂)ₙ₁-COOR₁ ; -(CH₂)ₙ₁-CO-NH-NH₂ ; -(CH₂)ₙ₁-H ; un halogène choisi parmi F, Cl, Br ou I ;
∘ un système cyclique ou hétérocyclique choisi parmi : 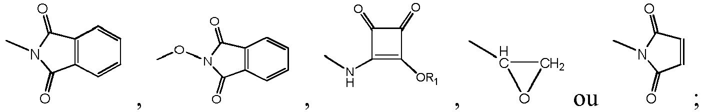 avec n₁, R₁ et Hal tels que définis à la revendication 1.

3. Composé selon la revendication 1 ou 2, **caractérisé en ce que** :
∘ le substituant L est choisi parmi :
∘∘ -CH=CH₂, -C=CH ou -NH₂ alors dans chacun de ces cas L₁ est absent,
∘∘ -CH₂- ; -CH₂-CH₂- ; -(CH₂)₃- ; -(CH₂)₄- ; -(CH₂)₅- ; -CH₂-CH₂-O-CH₂-CH₂- ; -(CH₂-CH₂-O)₂-CH₂-CH₂- ; -CH₂-CH₂-S-CH₂-CH₂- ; -CH₂-CH₂-S-CH₂-CH₂-O-CH₂-CH₂- ; 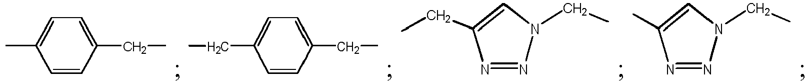 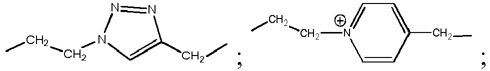 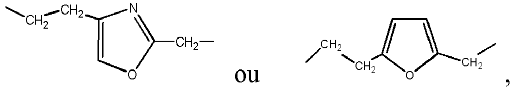 et
∘ le substituant L₁ est choisi parmi :
∘∘ -CH=CH₂ ; -C=CH ; -N₃ ; -SH ; -NH₂ ; -N=C=O ; -N=C=S ;-O-NH₂ ; -NHCO-CH₂Cl ; -COOH ; -COOR₁ ; -CO-NH-NH₂, un halogène choisi parmi F, Cl, Br ou I,
∘∘ un système cyclique ou hétérocyclique choisi parmi : 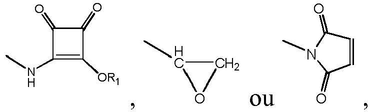 avec R₁ tel que défini à la revendication 1 ou 2.

4. Procédé de préparation d'un composé de formule (I) selon l'une quelconque des revendications 1 à 3 **caractérisé en ce que** le composé de départ utilisé est un composé répondant à la formule (II) : 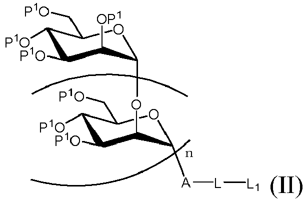 dans laquelle P¹, A, L, L₁ et n sont tels que définis à l'une quelconque des revendications 1 à 3.

5. Conjugué **caractérisé en ce qu'**il répond à la formule générale (III) : 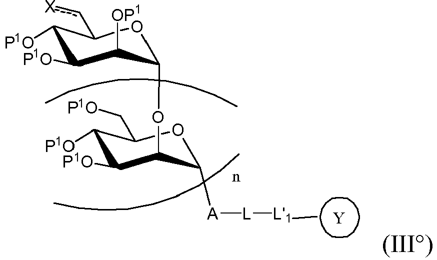
dans laquelle P¹, X, n, A et L sont tels que définis à l'une quelconque des revendications 1 à 3,
L₁' représente le substituant L₁ tel que défini à l'une quelconque des revendications 1 à 3 impliqué dans une liaison covalente avec un groupement fonctionnel porté par un composé d'intérêt Ⓨ, ledit composé d'intérêt Ⓨ étant choisi parmi des enzymes, des nanoparticules, des protéines, des anticorps ou des agents cytotoxiques.

6. Conjugué selon la revendication 5, **caractérisé en ce que** ledit conjugué présente une affinité IC₅₀ pour le récepteur du mannose 6-phosphate cation indépendant (RM6P-CI) d'au moins 10⁻⁵ M, et de préférence allant de 10⁻⁶ à 10⁻⁹ M.

7. Conjugué selon la revendication 5 ou 6, **caractérisé en ce que** le composé d'intérêt Ⓨ est une enzyme lysosomale ou une nanoparticule.

8. Conjugué selon l'une quelconque des revendications 5 à 7, pour son utilisation dans une méthode de traitement thérapeutique du corps humain ou animal, notamment choisie parmi l'enzymothérapie substitutive, la thérapie photodynamique ou le traitement du cancer.

9. Conjugué selon l'une quelconque des revendications 5 à 7, pour son utilisation dans une méthode de diagnostic, notamment de maladies ou d'affections associées à une augmentation ou une diminution de l'expression du RM6P-CI.

10. Utilisation d'au moins un composé de formule (I) tel que défini à l'une quelconque des revendications 2 à 3, pour former au moins une liaison covalente avec au moins un groupement fonctionnel porté par un composé d'intérêt Ⓨ, ledit composé d'intérêt Ⓨ étant choisi parmi des enzymes, des nanoparticules, des protéines, des anticorps ou des agents cytotoxiques.

11. Utilisation selon la revendication 10 de n' composé(s) de formule (I) pour former n' liaison(s) covalente(s) avec n' groupement(s) fonctionnel(s) du composé d'intérêt Ⓨ, avec n' étant un entier allant de 1 à 1000, et de préférence de 1 à 100.

12. Utilisation selon la revendication 10 ou 11 pour former le conjugué de formule générale (IIIbis) : 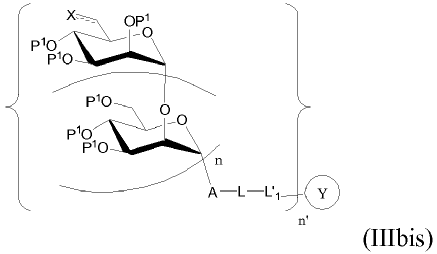
dans laquelle P¹, X, n, A, L, L'1 et Ⓨ sont tels que définis à la revendication 5,
n' est tel que défini à la revendication 11.

13. Procédé de préparation d'un conjugué de formule (III) tel que défini à la revendication 5 ou d'un conjugué de formule (IIIbis) tel que défini à la revendication 12, **caractérisé en ce que** l'on fait réagir :
- au moins un groupement fonctionnel porté par un composé d'intérêt Ⓨ, ledit composé d'intérêt étant choisi parmi des enzymes, des nanoparticules, des protéines, des anticorps ou des agents cytotoxiques, avec
- au moins un composé répondant à la formule (I) : 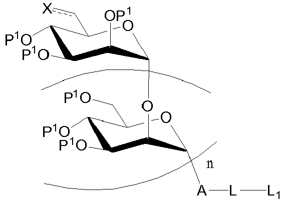 dans laquelle
P¹, X, A et n sont tels que définis à la revendication 1,
L et L₁ sont tels que définis à la revendication 2 ou 3.

## Patentansprüche

1. Verbindung, **gekennzeichnet durch** die allgemeine Formel (I) : 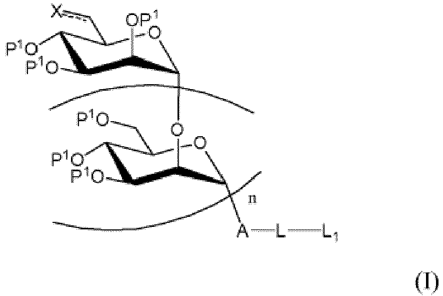 worin:
∘ n eine ganze Zahl von 1 bis 3 ist,
∘ ------- für eine Einfachbindung oder eine Doppelbindung steht,
∘ jedes P¹ jeweils unabhängig voneinander für H, eine Schutzgruppe, insbesondere ausgewählt aus (CH₃)₃Si-; tBuMe₂Si-; C₆H₅CH₂-; 4-CH₃OC₆H₅CH₂-; o-NO₂C₆H₅CH₂-; CH₃CO-; C₆H₅CO- oder CF₃CO-, steht;
∘ X für folgendes steht:
∘∘ wenn ------- für eine Einfachbindung steht:
∘∘∘ die Phosphonatgruppe: 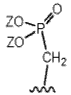
∘∘∘ die Fluorphosphonatgruppe: 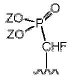
∘∘∘ die Bi-Fluorphosphonatgruppe: 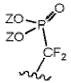
∘∘∘ die Carboxylatgruppe: 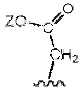
∘∘∘ die Malonatgruppe: 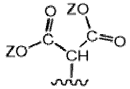
∘∘ wenn ------- für eine Doppelbindung steht:
∘∘∘ die Phosphonatgruppe: 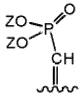
∘∘∘ die Carboxylatgruppe: 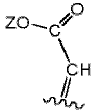
worin Z unabhängig voneinander für H; ein C₁-C₅-Alkyl; CF₃CH₂-; C₆H₅CH₂-; C₆H₅-; (CH₃)₃Si; ein Alkalimetall, ausgewählt aus Na, Li oder K; ein Ammonium NH₄ steht,
∘ A für einen zweiwertigen Rest steht, ausgewählt aus -O-, -S-, -NH-, -CH₂-,
∘ L für folgendes steht:
∘∘ -H; NH₂; - (CH₂)ₙ₁-CH=CH₂ oder -(CH₂)ₙ₁-C=CH, worin n eine ganze Zahl von 0 bis 4 ist, wobei dann in jedem dieser Fälle L₁ fehlt,
∘∘ einen gesättigten zweiwertigen Kohlenwasserstoffrest, der linear oder verzweigt ist, der substituiert oder unsubstituiert ist, der 1 bis 30 Kohlenstoffatome aufweist, einen ungesättigten zweiwertigen Kohlenwasserstoffrest, der linear oder verzweigt ist, der substituiert oder unsubstituiert ist, der 2 bis 30 Kohlenstoffatome aufweist.
∘∘ einen gesättigten oder ungesättigten zweiwertigen Kohlenwasserstoffrest, der wie oben definiert ist, worin eine oder mehrere -CH₂-, -CH=CH- und/oder -C=C-Gruppen des gesättigten oder ungesättigten Kohlenwasserstoffrests unabhängig voneinander ersetzt ist oder sind durch eine Gruppe -O-; -NH-; -NR₁-, worin R₁ für ein C₁-C₅-Alkyl steht; -S-; -CO-NH-; -NH-CO-O-;-O-N=CH-; -CO-NH-N=CH-; ein cyclisches oder heterocyclisches System, das gesättigt oder ungesättigt ist, das substituiert oder unsubstituiert ist;
∘ L₁ für folgendes steht:
-(CH₂)ₙ₁-CH=CH₂; -(CH₂)ₙ₁-C≡CH; -(CH₂)ₙ₁-N₃; -(CH₂)ₙ₁-SH;-(CH₂)ₙ₁-NH₂; -(CH₂)ₙ₁-N=C=O; -(CH₂)ₙ₁-N=C=S ; -(CH₂)ₙ₁-NHR₁ ; -(CH₂)ₙ₁-NR₁R₂; -(CH₂)ₙ₁-A₁-NH₂; -(CH₂)ₙ₁-A₁-NHR₁; -(CH₂)ₙ₁-A₁-NR₁R₂; -(CH₂)ₙ₁-NHCO-CH₂Hal; -(CH₂)ₙ₁-COZ₁; -(CH₂)ₙ₁-A₁COZ₁; -(CH₂)ₙ₁-O-N=CH₂; -(CH₂)ₙ₁-CO-NH-N=CH₂; -(CH₂)ₙ₁-H; ein cyclisches oder heterocyclisches System, das gesättigt oder ungesättigt ist, das substituiert oder unsubstituiert ist; ein Halogen, ausgewählt aus F, Cl, Br oder I;
worin
n₁ wie oben definiert ist,
R₁ und R₂ unabhängig voneinander für ein C₁-C₅-Alkyl stehen,
A₁ für -O-, -NH- steht,
Hal für Cl, Br oder I steht;
Z₁ für -OH, -OR, -NHR₁, -NH-NH₂, -NH-NHR₁, -NH-NR₁R₂,
worin R₁ und R₂ wie oben definiert sind, ein Halogen, ausgewählt aus F, Cl, Br oder I, steht.

2. Verbindung gemäß Anspruch 1, **dadurch gekennzeichnet, dass**:
L für folgendes steht:
∘ -NH₂, -(CH₂)ₙ₁-CH=CH₂ oder -(CH₂)ₙ₁-C≡CH, worin n₁ wie in Anspruch 1 definiert ist, wobei dann in jedem dieser Fälle L₁ fehlt,
∘ einen gesättigten zweiwertigen Kohlenwasserstoffrest, der linear oder verzweigt ist, der substituiert oder unsubstituiert ist, der 1 bis 10 Kohlenstoffatome aufweist, einen ungesättigten zweiwertigen Kohlenwasserstoffrest, der linear oder verzweigt ist, der substituiert oder unsubstituiert ist, der 2 bis 10 Kohlenstoffatomen aufweist.
∘ einen gesättigten oder ungesättigten zweiwertigen Kohlenwasserstoffrest, der wie oben definiert ist, worin eine oder mehrere -CH₂-, -CH=CH- und/oder -C≡C-Gruppen des gesättigten oder ungesättigten Kohlenwasserstoffrests unabhängig voneinander ersetzt ist oder sind durch:
∘∘ eine Gruppe -O-; -NH-; -S-; -CO-NH-; -NH-CO-O-; und/oder
∘∘ ein cyclisches oder heterocyclisches System, ausgewählt aus: 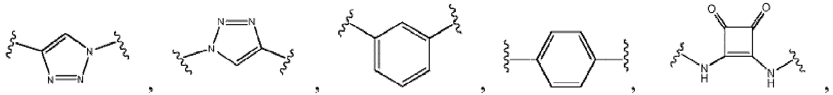 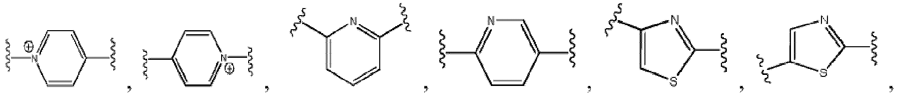 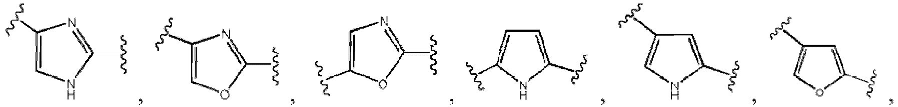 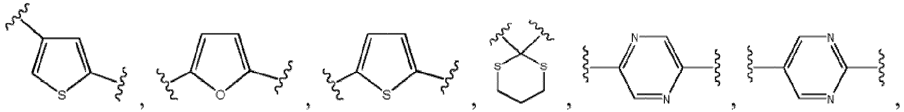 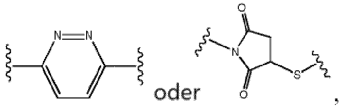
L₁ für folgendes steht:
-(CH₂)ₙ₁-CH=CH₂; -(CH₂)ₙ₁-C≡CH; -(CH₂)ₙ₁-N₃; -(CH₂)ₙ₁-SH; -(CH₂)ₙ₁-NH₂; -(CH₂)ₙ₁-N=C=O; -(CH₂)ₙ₁-N=C=S; -(CH₂)ₙ₁-O-NH₂; -(CH₂)ₙ₁-NHCO-CH₂Hal; -(CH₂)ₙ₁-COOH; -(CH₂)ₙ₁-COOR₁; -(CH₂)ₙ₁-CO-NH-NH₂; -(CH₂)ₙ₁-H; ein Halogen, ausgewählt aus F, Cl, Br oder I;
∘ ein cyclisches oder heterocyclisches System, ausgewählt aus: 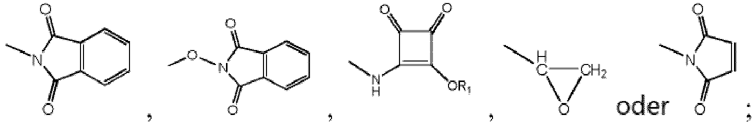 worin n₁, R₁ und Hal wie in Anspruch 1 definiert sind.

3. Verbindung gemäß Anspruch 1 oder 2, **dadurch gekennzeichnet, dass**:
∘ der Substituent L ausgewählt ist aus:
∘∘ -CH=CH₂, -C≡CH oder -NH₂, wobei dann in jedem dieser Fälle L₁ fehlt,
∘∘ -CH₂-; -CH₂-CH₂-; -(CH₂)₃-; -(CH₂)₄-; -(CH₂)₅-; -CH₂-CH₂-O-CH₂-CH₂-; -(CH₂-CH₂-O)₂-CH₂-CH₂-; -CH₂-CH₂-S-CH₂-CH₂-; -CH₂-CH₂-S-CH₂-CH₂-O-CH₂-CH₂-; 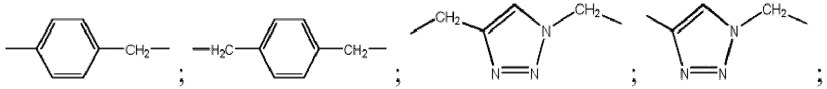 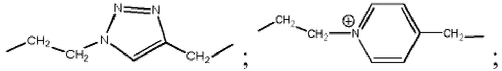 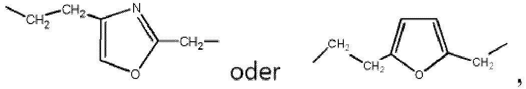 und
∘ der Substituent L₁ ausgewählt ist aus:
∘∘ -CH=CH₂; -C≡CH; -N₃; -SH; -NH₂; -N=C=O; -N=C=S; -O-NH₂; -NHCO-CH₂Cl; -COOH; -COOR₁; -CO-NH-NH₂, ein Halogen, ausgewählt aus F, Cl, Br oder I,
∘∘ einem cyclischen oder heterocyclischen System, ausgewählt aus: 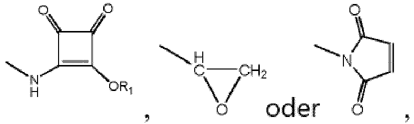 worin R₁ wie in Anspruch 1 oder 2 definiert ist.

4. Verfahren zur Herstellung einer Verbindung der Formel (I) gemäß einem der Ansprüche 1 bis 3, **dadurch gekennzeichnet, dass** als Ausgangsverbindung eine Verbindung der Formel (II) einsetzt wird: 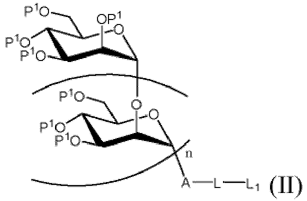 worin P¹, A, L, L₁ und n wie in einem der Ansprüche 1 bis 3 definiert sind.

5. Konjugat, **dadurch gekennzeichnet, dass** es der allgemeinen Formel (III) entspricht: 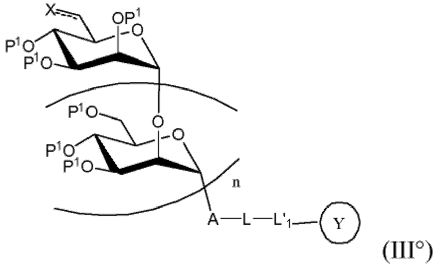
worin P¹, X, n, A und L wie in einem der Ansprüche 1 bis 3 definiert sind,
L₁' für den in einem der Ansprüche 1 bis 3 definierten Substituenten L₁ steht, der an einer kovalenten Bindung mit einer funktionellen Gruppe beteiligt ist, die von einer Verbindung von Interesse Ⓨ getragen wird, wobei die Verbindung von Interesse Ⓨ ausgewählt ist aus Enzymen, Nanoteilchen, Proteinen, Antikörpern oder Zytostatika.

6. Konjugat gemäß Anspruch 5, **dadurch gekennzeichnet, dass** das Konjugat eine IC₅₀-Affinität für den Kationenunabhängigen Mannose-6-Phosphat-Rezeptor (RM6P-CI) von mindestens 10⁻⁵ M und vorzugsweise 10⁻⁶ bis 10⁻⁹ M aufweist.

7. Konjugat gemäß Anspruch 5 oder 6, **dadurch gekennzeichnet, dass** die Verbindung von Interesse Ⓨ ein lysosomales Enzym oder ein Nanoteilchen ist.

8. Konjugat gemäß einem der Ansprüche 5 bis 7 zur Verwendung in einem Verfahren zur therapeutischen Behandlung des menschlichen oder tierischen Körpers, insbesondere ausgewählt aus Enzymersatztherapie, photodynamischer Therapie oder Krebsbehandlung.

9. Konjugat gemäß einem der Ansprüche 5 bis 7 zur Verwendung in einem diagnostischen Verfahren, insbesondere von Krankheiten oder Zuständen, die mit einer Zunahme oder Abnahme der Expression von RM6P-CI verbunden sind.

10. Verwendung von mindestens einer wie in einem der Ansprüche 2 bis 3 definierten Verbindung der Formel (I) zur Bildung mindestens einer kovalenten Bindung mit mindestens einer funktionellen Gruppe, die von einer Verbindung vom Interesse Ⓨ getragen wird, wobei die Verbindung von Interesse Ⓨ ausgewählt ist aus Enzymen, Nanoteilchen, Proteinen, Antikörpern oder Zytostatika.

11. Verwendung gemäß Anspruch 10 von n' Verbindung(en) der Formel (I) zur Bildung von n' kovalenten Bindung(en) mit n' funktionellen Gruppen der Verbindung von Interesse Ⓨ, wobei n' eine ganze Zahl von 1 bis 1000 und vorzugsweise von 1 bis 100 ist.

12. Verwendung gemäß Anspruch 10 oder 11 zur Bildung des Konjugats der allgemeinen Formel (IIIbis): 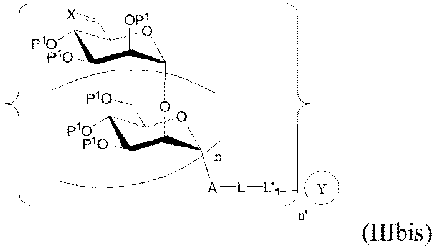 worin P¹, X, n, A, L, L'1 und Ⓨ wie in Anspruch 5 definiert sind, n' wie in Anspruch 11 definiert ist.

13. Verfahren zur Herstellung eines wie in Anspruch 5 definierten Konjugats der Formel (III) oder eines wie in Anspruch 12 definierten Konjugats der Formel (IIIbis), **dadurch gekennzeichnet, dass** folgendes umgesetzt wird:
- mindestens eine funktionelle Gruppe, die von einer Verbindung von Interesse Ⓨ getragen wird, wobei die Verbindung von Interesse ausgewählt ist aus Enzymen, Nanoteilchen, Proteinen, Antikörpern oder Zytostatika, mit
- mindestens einer Verbindung, die der Formel (I) entspricht: 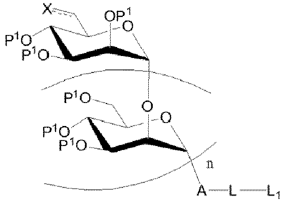 worin
P¹, X, A und n wie in Anspruch 1 definiert sind,
L und L₁ wie in Anspruch 2 oder 3 definiert sind.

## Claims

1. A compound **characterized in that** it has the general formula (I): 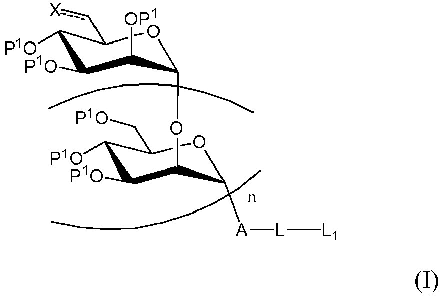 in which:
∘ n is an integer ranging from 1 to 3,
∘ ------- represents a single bond or a double bond,
∘ each of the P¹ represents, independently of one another, H, or a protecting group chosen from (CH₃)₃Si-; tBuMe₂Si-; C₆H₅CH₂-; 4-CH₃OC₆H₅CH₂-; o-NO₂C₆H₅CH₂-; CH₃CO-; C₆H₅CO- or CF₃CO-;
∘ X represents:
∘∘ when ------- is a single bond:
∘∘∘ the phosphonate group: 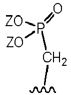
∘∘∘ the fluorophosphonate group: 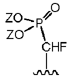
∘∘∘ the difluorophosphonate group: 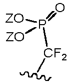
∘∘∘ the carboxylate group: 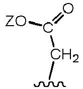
∘∘∘ the malonate group: 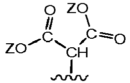
∘∘ when ------- is a double bond:
∘∘∘ the phosphonate group: 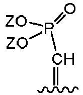
∘∘∘ the carboxylate group: 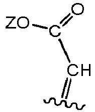 with Z representing, independently of one another, H; a C₁-C₅ alkyl; CF₃CH₂-; C₆H₅CH₂-; C₆H₅-; (CH₃)₃Si-; an alkali metal chosen from Na, Li or K; an ammonium NH₄;
∘ A represents a divalent radical chosen from -O-, -S-, -NH-, -CH₂-;
∘ L represents:
∘∘ -H; -NH₂; -(CH₂)ₙ₁-CH=CH₂ or -(CH₂)ₙ₁-C≡CH with n₁ representing an integer ranging from 0 to 4, then in each of these cases, L₁ is absent,
∘∘ a substituted or unsubstituted, linear or branched, saturated divalent hydrocarbon-based radical having from 1 to 30 carbon atoms, a substituted or unsubstituted, linear or branched, unsaturated divalent hydrocarbon-based radical having from 2 to 30 carbon atoms,
∘∘ a saturated or unsaturated divalent hydrocarbon-based radical as defined above, in which one or more -CH₂-, -CH=CH- and/or -C≡C- groups of the saturated or unsaturated hydrocarbon-based radical is (are) replaced, independently of one another, with an -O- group; -NH- group; -NR₁- group with R₁ representing a C₁-C₅ alkyl; -S- group; -CO-NH- group;-NH-CO-O- group; -O-N=CH- group; -CO-NH-N=CH- group; a substituted or unsubstituted, saturated or unsaturated, cyclic or heterocyclic system;
∘ L₁ represents:
-(CH₂)ₙ₁-CH=CH₂; -(CH₂)ₙ₁-C≡CH; -(CH₂)ₙ₁-N₃; -(CH₂)ₙ₁-SH; -(CH₂)ₙ₁-NH₂; -(CH₂)ₙ₁-N=C=O; -(CH₂)ₙ₁-N=C=S; -(CH₂)ₙ₁-NHR₁; -(CH₂)ₙ₁-NR₁R₂; -(CH₂)ₙ₁-A₁-NH₂; -(CH₂)ₙ₁-A₁-NHR₁; -(CH₂)ₙ₁-A₁-NR₁R₂; -(CH₂)ₙ₁-NHCO-CH₂Hal; -(CH₂)ₙ₁-COZ₁; -(CH₂)ₙ₁-A₁COZ₁; -(CH₂)ₙ₁-O-N=CH₂; -(CH₂)ₙ₁-CO-NH-N=CH₂; -(CH₂)ₙ₁-H; a substituted or unsubstituted, saturated or unsaturated, cyclic or heterocyclic system; a halogen chosen from F, Cl, Br or I; with
n₁ as defined above,
R₁ and R₂ representing, independently of one another, a C₁-C₅ alkyl,
A₁ representing -O-, -NH-,
Hal representing Cl, Br or I;
Z₁ representing -OH, -OR₁, -NHR₁, -NH-NH₂, -NH-NHR₁, -NH-NR₁R₂, with R₁ and R₂ as defined above, a halogen chosen from F, Cl, Br or I.

2. The compound as claimed in claim 1, **characterized in that**:
L represents:
∘ -NH₂, -(CH₂)ₙ₁-CH=CH₂ or -(CH₂)ₙ₁-C≡CH, with n₁ as defined in claim 1, then in each of these cases, L₁ is absent,
∘ a substituted or unsubstituted, linear or branched, saturated divalent hydrocarbon-based radical having from 1 to 10 carbon atoms, a substituted or unsubstituted, linear or branched, unsaturated divalent hydrocarbon-based radical having from 2 to 10 carbon atoms,
∘ a saturated or unsaturated, divalent hydrocarbon-based radical as defined above, in which one or more -CH₂-, -CH=CH- and/or -C≡C- groups of the saturated or unsaturated hydrocarbon-based radical is (are) replaced, independently of one another, with:
∘∘ an -O- group; -NH- group; -S- group; -CO-NH- group; -NH-CO-O- group; and/or
∘∘ a cyclic or heterocyclic system chosen from: 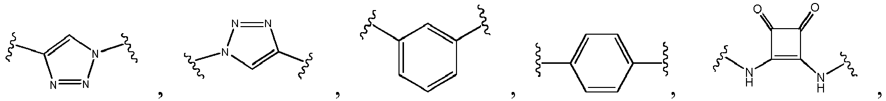 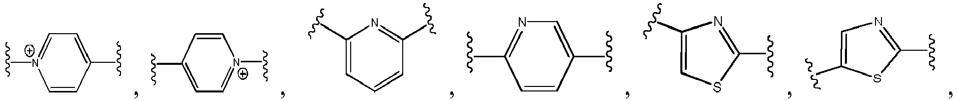 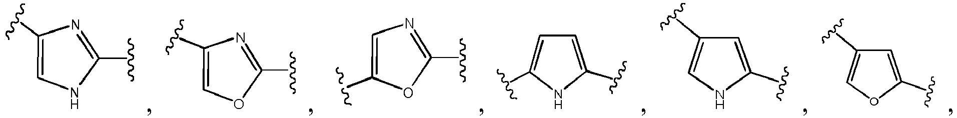 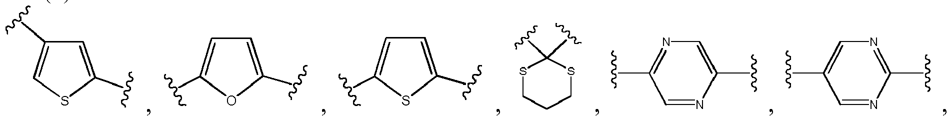 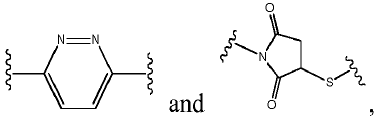
L₁ represents:
-(CH₂)ₙ₁-CH=CH₂; -(CH₂)ₙ₁-C≡CH; -(CH₂)ₙ₁-N₃; -(CH₂)ₙ₁-SH; -(CH₂)ₙ₁-NH₂; -(CH₂)ₙ₁-N=C=O; -(CH₂)ₙ₁-N=C=S; -(CH₂)ₙ₁-O-NH₂; -(CH₂)ₙ₁-NHCO-CH₂Hal; -(CH₂)ₙ₁-COOH; -(CH₂)ₙ₁-COOR₁; -(CH₂)ₙ₁-CO-NH-NH₂; -(CH₂)ₙ₁-H; a halogen chosen from F, Cl, Br or I;
∘ a cyclic or heterocyclic system chosen from: 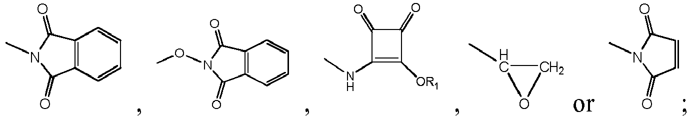 with n₁, R₁ and Hal as defined in claim 1.

3. The compound as claimed in claim 1 or 2, **characterized in that**:
∘ the substituent L is chosen from:
∘∘ -CH=CH₂, -C≡CH or -NH₂, then in each of these cases, L₁ is absent;
∘∘ -CH₂-; -CH₂-CH₂-; -(CH₂)₃-; -(CH₂)₄-; -(CH₂)₅-; -CH₂-CH₂-O-CH₂-CH₂-; -(CH₂-CH₂-O)₂-CH₂-CH₂-; -CH₂-CH₂-S-CH₂-CH₂-; -CH₂-CH₂-S-CH₂-CH₂-O-CH₂-CH₂- ; 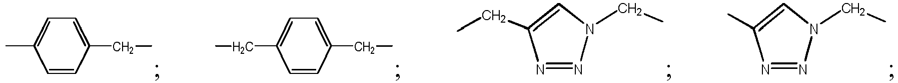 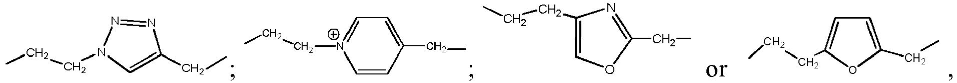 and
∘ the substituent L₁ is chosen from:
∘∘ -CH=CH₂; -C≡CH; -N₃; -SH; -NH₂; -N=C=O; -N=C=S;-O-NH₂; -NHCO-CH₂Cl; -COOH; -COOR₁; -CO-NH-NH₂, a halogen chosen from F, Cl, Br or I,
∘∘ a cyclic or heterocyclic system chosen from: 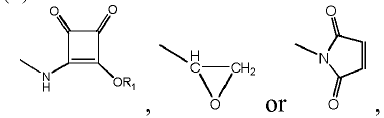 with R₁ as defined in claim 1 or 2.

4. A process for preparing a compound of formula (I) as claimed in any one of claims 1 to 3, **characterized in that** the starting compound used is a compound corresponding to the formula (II): 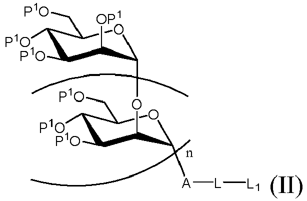 in which P¹, A, L, L₁ and n are as defined in any one of claims 1 to 3.

5. A conjugate **characterized in that** it corresponds to general formula (III): 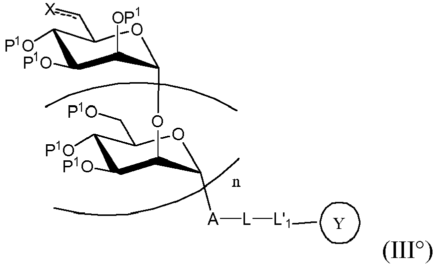
in which P¹, X, n, A and L are as defined in any one of claims 1 to 3,
L'₁ represents the substituent L₁ as defined in any one of claims 1 to 3 involved in a covalent bond with a functional group borne by a compound of interest Ⓨ, said compound of interest Ⓨ being chosen from enzymes, nanoparticles, proteins, antibodies or cytotoxic agents.

6. The conjugate as claimed in claim 5, **characterized in that** said conjugate has an IC₅₀ affinity for the cation-independent mannose 6-phosphate receptor (CI-M6PR) of at least 10⁻⁵ M, and preferably ranging from 10⁻⁶ to 10⁻⁹ M.

7. The conjugate as claimed in claim 5 or 6, **characterized in that** the compound of interest Ⓨ is a lysosomal enzyme or a nanoparticle.

8. The conjugate as claimed in any one of claims 5 to 7, for use thereof in a method for therapeutic treatment of the human or animal body, in particular chosen from enzyme replacement therapy, photodynamic therapy or cancer treatment.

9. The conjugate as claimed in any one of claims 5 to 7, for use thereof in a method of diagnosis, in particular of diseases or ailments associated with an increase or a decrease in CI-M6PR expression.

10. The use of at least one compound of formula (I) as defined in either one of claims 2 and 3, for forming at least one covalent bond with at least one functional group borne by a compound of interest Ⓨ, said compound of interest Ⓨ being chosen from enzymes, nanoparticles, proteins, antibodies or cytotoxic agents.

11. The use as claimed in claim 10, of n' compound(s) of formula (I) for forming n' covalent bond(s) with n' functional group(s) of the compound of interest Ⓨ, with n' being an integer ranging from 1 to 1000, and preferably from 1 to 100.

12. The use as claimed in claim 10 or 11, for forming the conjugate of general formula (IIIa): 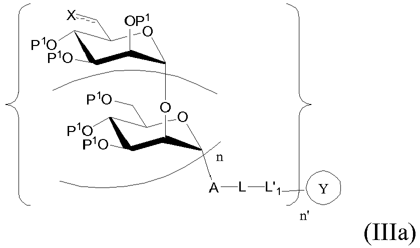
in which P¹, X, n, A, L, L'₁ and Ⓨ are as defined in claim 5,
n' is as defined in claim 11.

13. A process for preparing a conjugate of formula (III) as defined in claim 5 or a conjugate of formula (IIIa) as defined in claim 12, **characterized in that**:
- at least one functional group borne by a compound of interest Ⓨ, said compound of interest being chosen from enzymes, nanoparticles, proteins, antibodies or cytotoxic agents, is reacted with
- at least one compound corresponding to the formula (I): 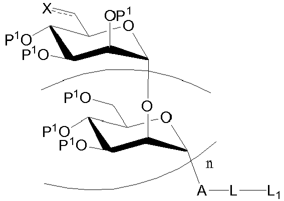 in which
P¹, X, A and n are as defined in claim 1,
L and L₁ are as defined in claim 2 or 3.
